Europäisches Patentamt

European Patent Office

Office européen des brevets

(11) Numéro de publication: **0 229 537**
**A1**

## (12) DEMANDE DE BREVET EUROPEEN

(21) Numéro de dépôt: 86402375.9

(22) Date de dépôt: 23.10.86

(51) Int. Cl.4: **A61H 1/02** , A63B 29/00 , A61H 3/00

(30) Priorité: 30.10.85 FR 8516097

(43) Date de publication de la demande:
22.07.87 Bulletin 87/30

(84) Etats contractants désignés:
AT BE CH DE ES GB GR IT LI LU NL SE

(71) Demandeur: **Chareire, Jean-Louis**
**66, rue Aristide Briand**
**F-92300 Levallois Perret(FR)**

(72) Inventeur: **Chareire, Jean-Louis**
**66, rue Aristide Briand**
**F-92300 Levallois Perret(FR)**

(74) Mandataire: **Bonnetat, Christian et al**
**Cabinet PROPI Conseils 23 rue de Léningrad**
**F-75008 Paris(FR)**

(54) **Appareil d'assistance mécanique de la propulsion par les jambes.**

(57) Appareil d'assistance mécanique de la propulsion par les jambes.

L'invention concerne le domaine de la mobilité individuelle de l'être humain.

L'appareil selon l'invention comprend une selle - (1), généralement munie d'un moyen (2) de fixation aux hanches. Une articulation (11) de la selle, permet le mouvement pendulaire de deux dispositifs téléscopiques (3) dont la force d'extension provient d'un ressort comprimé ou d'un fluide sous pression généré, par exemple, par un groupe moto-compresseur (7). Un bras de junction (5) transmet le mouvement à une articulation (6) fixée à l'avant de la chaussure. Une articulation (4) déblocable automatiquement en fin de foulée propulsive permet la remontée du bras (5) et du pied. Un système de pilotage automatique synchronise l'effort moteur avec l'effort musculaire d'extension de la jambe et du pied.

Application à tous les actes nécessitant le mouvement de propulsion par les jambes, d'un individu capable ou non d'un effort musculaire intense.

Fig. 2

EP 0 229 537 A1

## Appareil d'assistance mécanique de la propulsion par les jambes.

La présente invention concerne le domaine de la mobilité individuelle de l'être humain et plus particulièrement l'assistance mécanique de la propulsion par les jambes.

La propulsion par les jambes, qui fait appel aux muscles situés au niveau du bassin et des jambes conditionne notamment tous les déplacements terrestres non motorisés de l'homme ce qui comprend la possibilité de :

Marcher, courir, bondir, monter ou descendre une pente.

Cette propulsion par les jambes permet aussi l'usage d'accessoires favorisant le déplacement sur divers terrains ou sur l'eau qui sont par exemple :

Les skis, patins à glace, patins à roulettes, bicyclettes et divers engins terrestres ou nautiques à propulsion par pédalage ou moyen apparenté.

Enfin, les muscles servant à la propulsion par les jambes permettent aussi à l'individu de demeurer dans une position de fléchissement statique des membres inférieurs, pour effectuer certains travaux par exemple.

Le but de l'invention, qui est un appareil d'assistance mécanique de la propulsion par les jambes, est donc d'augmenter les performances naturelles de l'individu pour tous les usages précédemment cités. L'appareil agit comme un renforcement du système propulseur naturel de l'homme tout en laissant à celui-ci la possibilité d'un usage extrêmement varié de cette force accrue.

Les recherches effectuées pour déterminer l'art antérieur éventuel ont fait apparaître les deux catégories d'inventions suivantes:

D'une part des chaussures surélevées possédant des ressorts sous la semelle, ou des échasses à ressort. D'autre part, un certain nombre de prothèses pour les membres inférieurs destinées à jouer le rôle de squelette ou, exceptionnellement, destinées à permettre la marche lente à des paraplégiques.

Les chaussures ou échasses à ressorts diffèrent fondamentalement de l'invention en objet, d'abord parce qu'elles ne produisent que l'amplification d'un mouvement naturel et non pas son assistance mécanique en parallèle. En effet, l'accroissement de force qu'elles peuvent produire est uniquement transmis au corps par le squelette des jambes. Ensuite, ces chaussures ou échasses à ressort, qui surélèvent notablement le pied perturbent l'équilibre naturel.

Les prothèses visant à remplacer le squelette manquant d'un membre inférieur ne constituent pas des assistances à la propulsion. Enfin, les prothèses destinées à permettre la marche aux paraplégiques ont une géométrie inspirée de celle du squelette et qui comprend notamment l'articulation du genou. Cette disposition, totalement différente de celle de l'appareil selon l'invention est très peu favorable à l'exercice des forces très élevées qui sont nécessaires à la propulsion verticale de l'individu et aboutit à un appareil lourd et encombrant.

La forme de l'appareil objet de l'invention est conçue au contraire pour l'exercice rapide sur le corps de forces ascendantes importantes, mais il s'applique à des individus normaux ou diminués physiques qui ont la possibilité de mouvoir leurs pieds pendant un déplacement et d'exercer leur équilibre naturel.

Par ailleurs, le pied reposant sur le sol, et l'appareil n'entravant aucun mouvement naturel, celui-ci ne perturbe pas les conditions d'équilibre de l'individu. De plus, l'appareil permet l'usage cumulé d'accessoires tels que skis, patins, etc... sans que l'équilibre naturel, adapté par apprentissage à chacun de ces usages ne soit modifié.

Les domaines d'application de l'invention sont nombreux et variés et on peut citer, à titre d'exemples non limitatifs :

-Tous déplacements individuels à buts sportifs, utilitaires ou touristiques pour individus à musculature normale :

. Sans accesssoires complémentaires, mouvements en terrains rocheux, sabloneux, marécageux, montagneux non carrossable ou à très forte pente, escaliers, et tous endroits où il est nécessaire de bondir parmi des obstacles et de gravir de longues et fortes pentes.

. Avec des skis munis pour usage temporaire en côte d'un revêtement interdisant le glissement vers l'arrière, tel que les peaux de phoque, possibilité de gravir les plus fortes pentes sans fatigue. Avec des patins à glace, traversée rapide d'étendues gelées et lisses. Avec une bicyclette sans sa selle initiale ou autre engin terrestre à propulsion par pédalage ou moyen apparenté ou des patins à roulettes de diamètre suffisant, accomplissement de déplacements routiers sur distances moyennes. L'emport d'accessoires démontables et légers permet donc d'accomplir un déplacement sur un parcours très évolutif, accessible ou non aux roues. Enfin, il est possible d'effectuer des déplacements nautiques avec un bateau à hélice ou à roues dont l'énergie est distribuée sur un pédalier ou mécanisme analogue.

-Déplacements individuels des diminués physiques :

. Aide au déplacement des personnes à très faible force musculaire avec possibilité d'accès aux rubriques précédentes.

. Aide aux infirmes partiels tels que les individus atteints d'affections graves du système cardio-vasculaire ou souffrant de séquelles de la poliomyélite par exemple, leur permettant de monter ou descendre de fortes pentes non accessibles aux chariots d'infirmes ou des escaliers. Assistance possible à la colonne vertébrale par soutien supplémentaire aux épaules et possibilité de soutien partiel par les mains. Aide à la station debout.

-Aide au travail :

. Assistance à la montée ou descente des pentes non carrossables ou escaliers dans l'industrie du bâtiment et des travaux publics dans certaines mines etc ...

. Transport à dos d'homme de fortes charges en terrains variés.

. Remorquage par un homme, de charges ou d'outils en terrain non carrossable.

. Soutien statique en flexion des jambes pour les peintres, mécaniciens, etc ...

-Jeu sportif :

. La pratique de cet appareil pour la marche bondissante ou l'ascension rapide procure à ses adeptes une sensation combinant celle que l'on peut ressentir sur la selle d'un cheval vigoureux et sur celle d'une moto de cross.

Toutes ces applications sont donc susceptibles d'améliorer la qualité de vie d'un grand nombre d'individus et de représenter un débouché industriel important.

Ces objectifs sont atteints avec le système selon l'invention notamment grâce aux moyens suivants :

L'appareil est muni d'une selle à laquelle aboutissent les efforts de poussée ascendante et qui les transmet au corps. Cette selle, qui pour la plupart des usages n'est pas en permanence l'objet de poussées ascendantes, comprend un dispositif de fixation aux hanches de l'individu, fermé par une boucle de ceinture, qui lui permet d'exercer toujours une certaine force minimum d'appui sous les fesses afin de conserver un très bon contact à tout moment. Dans certains cas, la fixation aux hanches peut être remplacée ou complétée par une fixation au-dessus des épaules.

Une articulation à axe sensiblement perpendiculaire au plan médian du corps est fixée sous la selle et deux dispositifs téléscopiques lui sont reliés symètriquement par rapport au plan médian de manière à ce qu'ils puissent chacun effectuer un mouvement pendulaire sous la selle autour de cet axe.

La forme des dispositifs téléscopiques et l'emplacement de l'articulation sous la selle permettent à ceux-ci d'effetuer leurs déplacements pendulaires accompagnant ceux des jambes sans se heurter à l'encombrement extérieur de l'arrière et de l'intérieur de celles-ci.

La disposition à l'arrière des jambes de l'individu est en effet la moins encombrante et la plus facilement compatible avec les mouvements naturels. Les dispositifs téléscopiques comprennent de plus, au voisinage de leur articulation sur la selle, une articulation complémentaire d'axe sensiblement parallèle au plan médian du corps et qui permet, le cas échéant, l'écartement latéral des jambes.

Les articulations à axe sensiblement perpendiculaire ou sensiblement parallèle au plan médian qui sont situées dans la zône de la selle et reliées aux dispositifs téléscopiques sont placées de préférence dans cet ordre en allant de la selle vers les dispositifs téléscopiques mais l'ordre inverse est aussi du domaine de l'invention. On entend par les termes, sensiblement parallèle, ou sensiblement perpendiculaire, utilisés pour définir un certain nombre de directions caractéristiques de l'invention, que les directions réellement choisies pour des commodités technologiques, qui peuvent s'écarter jusqu'à 15 ou 20° par exemple, de la pure direction parallèle ou perpendiculaire mais sans entraîner une modification du rôle fondamental de l'articulation concernée, sont du domaine de l'invention.

Les fonctions que doivent remplir les dispositifs téléscopiques sont, d'une part l'exercice d'une force pendant leur extension et d'autre part, de résister au couple qui résulte du fait que la force n'est pas exercée dans l'axe du téléscope mais s'applique à l'extrêmité du bras de jonction avec le pied. Le dispositif téléscopique peut donc comprendre une pièce mâle et une pièce femelle rectilignes ou curvilignes coulissant l'une dans l'autre, le coulissement s'effectuant par l'intermédiaire d'un système de roulettes, ou de billes ou tout autre procédé comportant éventuellement une cage intermédiaire, capable d'éviter le coincement lors du coulissement sous l'effet du couple. En variante, le dispositif téléscopique peut comprendre à la place des roulettes ci-dessus, une barre rigide et trois articulations supplémentaires dont l'ensemble libère le téléscope des effets du couple, au prix d'un encombrement plus important.

On peut placer autour de l'articulation de la selle à axe sensiblement perpendiculaire au plan médian un groupe de trois engrenages coniques dont deux sont solidaires des mouvements pendulaires des dispositifs téléscopiques et l'axe du troisième est dans le plan médian et fixé à la selle,ce qui stabilise angulairement celle-ci par rapport à la bissectrice des jambes.

A partir de la selle, les dispositifs téléscopiques descendent vers le sol en restant de préférence peu éloignés l'un de l'autre, les jambes étant serrées, de sort qu'ils demeurent entièrement ou presqu'entièrement à l'arrière des jambes et le plus près possible de celles-ci sans jamais les heurter.

L'extrêmité inférieure des dispositifs téléscopiques, donc celle qui est opposée aux articulations sous la selle, est reliée par une articulation à axe sensiblement perpendiculaire au plan médian du corps à un bras de jonction qu'une autre articulation, sensiblement parallèle à la précédente relie à la partie avant de la chaussure. La forme du bras est telle qu'elle lui permet d'effectuer des déplacements angulaires importants autour de ses deux articulations, de préférence sans heurter la chaussure, la cheville ou la partie inférieure du mollet.

L'articulation qui relie le bras de jonction au bas du dispositif téléscopique est munie d'un rochet qui autorise l'abaissement individuel du pied et du bras de jonction jusqu'à mise en butée de celui-ci, mais qui interdit, à la remontée du pied, la remontée du bras de jonction indépendamment de celle du dispositif téléscopique concerné. Ce rochet peut être mis hors service par un moyen électrique ou hydraulique sur l'ordre d'un signal hydraulique ou électrique générés par l'arrivée en position d'extension maximale du dispositif téléscopique, uniquement lorsque cette extension maximale a lieu pour une position du pied en arrière du corps, donc à la fin de la phase de propulsion. Ainsi, à la fin de cette phase, le pied peut remonter ce qui lui permet de repasser devant l'autre pied qui est au sol, sans heurter le sol. La distance, mesurée lorsque l'individu est debout sans talons, entre la base du dispositif téléscopique et un sol plat, est de préférence suffisante pour qu'il n'y ait pas de choc direct avec le sol pendant certaines phases de la course ou du bondissement ou la descente de pentes, escaliers, etc ...

Le dispositif téléscopique est muni de moyens internes, pistons, cylindres, etc ... qui lui permettent d'exercer une force importante accompagnant son extension, sous l'effet d'un ressort préalablement comprimé ou de la pression d'un fluide. Si l'articulation inférieure du dispositif téléscopique est bloquée par son rochet, cette force se transmet d'une part à l'extrémité du bras de jonction, donc à la partie avant de la chaussure, et d'autre part, à la selle par son articulation.

L'exercice de cette force est synchronisé avec l'ensemble des efforts musculaires produisant l'extension de la jambe et du pied, soit naturellement si la force est due à la détente d'un ressort comprimé dans la partie avant de la foulée, soit par un système de pilotage fondé sur la mesure d'une position angulaire des dispositifs téléscopiques pour l'admission, et sur la détection de leur extension maximale pour la vidange, si la force est due à une pression de fluide provenant d'un générateur.

Le générateur de fluide sous pression est en général un groupe moto-compresseur portable par l'individu ou d'autres moyens de génération autonomes mais cela peut être aussi un réservoir portable ou un tuyau souple relié à une source fixe d'air comprimé pour les usages à court rayon d'action.

L'invention peut être munie, en plus des dispositifs précédents, d'un système d'aide mécanique au mouvement pendulaire des jambes. Pour cela, un point voisin du sommet de chaque dispositif téléscopique est relié à un vérin téléscopique qui prend appui derrière la selle sur une articulation liée à celle-ci de façon suffisamment rigide. Le vérin téléscopique est en général alimenté par un fluide comprimé, simultanément avec le dispositif téléscopique auquel il n'est pas mécaniquement relié.

L'appareil peut comprendre d'autres points d'appui sur le corps que la selle. Il peut en effet posséder des poignées latérales parallèles, reliées à l'arrière de la selle, qui permettent lorsque l'intéressé a les bras tendus le long du corps, de recevoir un important effort vertical ascendant dans les bras. Ces poignées permettent également d'éviter de fixer l'appareil au corps dans certains cas, celui-ci étant alors maintenu en position par les bras. Il peut exister aussi un dispositif de transmission de l'effort ascendant reçu par la selle jusqu'aux épaules grâce à un système de pièces de transmission d'effort, et d'appui sous les bras, reliés par des articulations éventuellement blocables.

Ainsi, dans ses éléments essentiels, l'innovation concerne un appareil d'assistance mécanique de la propulsion par les jambes s'appliquant, sans altération des conditions d'équilibre de l'individu, à la marche, à la course, au bondissement, à la montée ou descente des pentes et au maintien en position de fléchissement statique sur les jambes, l'individu qui en est muni pouvant aussi être équipé d'accessoires favorisant la mobilité tels que skis, patins à glace ou à roulettes, bicyclettes et divers engins terrestres ou nautiques à propulsion par pédalage ou moyen apparenté, caractérisé en ce que :

Il comprend une selle, généralement munie de moyens qui permettent sa fixation au moins sur les hanches, comportant au moins une articulation à laquelle sont reliées les extrêmités supérieures de deux dispositifs téléscopiques, symètriques par rapport audit plan médian, descendant vers le sol en respectant une garde suffisante et en demeurant de préférence constamment derrière les jambes et le plus près possible de celles-ci sans entraver leurs mouvements. Au voisinage de l'extrêmité inférieure de chaque dispositif téléscopique est fixée une articulation munie d'un moyen de blocage pouvant être mis hors service vers la fin de la foulée propulsive de l'individu et généralement pour au moins la durée de la remontée du pied concerné. Cette articulation est reliée à un bras de jonction qu'une autre articulation relie à la partie avant de la chaussure ou à un accessoire fixé à celle-ci. La forme dudit bras de jonction lui permet d'effectuer des déplacements angulaires suffisants autour de ses deux articulations de préférence sans heurter la chaussure, la cheville ou le bas du mollet. Les dispositifs téléscopiques sont munis de moyens pour exercer, simultanément avec l'effort musculaire d'extension de la jambe et du pied, une force due à un ressort comprimé ou à la pression d'un fluide qui, lorsque leur articulation inférieure est bloquée, tend à éloigner l'articulation de la selle de celle de la chaussure concernée, ce qui provoque une aide mécanique à l'effort musculaire.

L'invention sera mieux comprise par la description détaillée d'exemples de réalisations, cette description s'appuyant sur les figures en annexe qui représentent :

Figure 1 : Vue de face d'un homme équipé de l'appareil selon l'invention.

Figure 2 : Vue de profil d'un homme équipé de l'appareil

Figure 3 : Vue de dos d'un homme équipé de l'appareil

Figure 4 : Vue de profil d'un homme équipé de l'appareil, montrant un emplacement possible pour un générateur de fluide sous pression.

Figure 5 : Vue de profil de l'appareil selon l'invention, seul et à l'état replié pour le transport ou le rangement.

Figure 6 : Vue de profil d'une selle équipée d'un premier type de dispositif de fixation sur les hanches.

Figure 7: Vue de dos de cette selle et de son dispositif de fixation.

Figure 8 : Vue de dessus de cette selle et de son dispositif de fixation.

Figure 9 : Vue de profil d'une selle équipée d'un second type de fixation sur les hanches.

Figure 10 : Vue de dessus de cette même selle.

Figure 11 : Vue de dos de cette même selle et de son dispositif de fixation.

Figure 12 : Vue de profile d'un homme muni de l'appareil selon l'invention au moment où sa jambe avant atterrit sur le sol après un bond.

Figures 13 et 14 : Vues de profil successives chronologiquement après l'atterrissage représenté sur la figure 12.

Figure 15 : Vue succédant chronologiquement à la vue 14, au moment où les deux dispositifs téléscopiques sont dans le même plan de profil.

Figure 16 : Vue de profil de l'intéressé au moment où il va bondir.

Figure 17 : Vue de profile de l'intéressé pendant le bond.

Figure 18 : Vue de profil d'une homme muni de l'appareil pour grimper une forte côte, au moment où le poids de son corps vient de se porter sur la jambe amont.

Figure 19 : Vue suivant chronologiquement la figure 18.

Figure 20: Vue suivant chronologiquement la figure 19. La propulsion par la jambe arrière est presque terminée et l'intéressé vient de poser la jambe amont sans y avoir encore porté le poids de son corps.

Figure 21 : Coupe selon A de la partie centrale de la selle vue de l'arrière montrant un premier mode de réalisation des articulations supérieures des dispositifs téléscopiques.

Figure 22 : Coupe selon C-C montrant la selle de profil avec le premier mode de réalisation des articulations supérieures des dispositifs téléscopiques.

Figure 23 : Coupe selon B-B montrant la selle vue de dessus avec le premier mode de réalisation des articulations supérieures des dispositifs téléscopiques.

Figure 24 : Vue de dessus d'un dispositif téléscopique montrant le système de butée établi sur une articulation supérieure de celui-ci dans le cas où il s'agit du premier mode de réalisation.

Figure 25 : Coupe selon D de la partie centrale de la selle, vue de l'arrière, montrant un second mode de réalisation des articulations supérieures des dispositifs téléscopiques.

Figure 26 : Coupe selon F-F montrant la selle de profil avec le second mode de réalisation des articulations supérieures des dispositifs téléscopiques.

Figure 27 : Coupe selon E-E montrant la selle vue de dessus avec le second mode de réalisation des articulations supérieures des dispositifs téléscopiques.

Figure 28 : Vue par l'arrière de la selle du sommet des deux dispositifs téléscopiques montrant le système de butée établi sur une des articulations supérieures de ceux-ci dans le cas où il s'agit du second mode de réalisation.

Figure 29 : Coupe en vue de profil d'un premier dispositif téléscopique.

Figure 30 : Coupe en vue de dessus selon son axe longitudinal du premier dispositif téléscopique montrant les roulettes supérieures.

Figure 31 : Coupe en vue de dessus du premier dispositif téléscopique montrant les roulettes inférieures.

Figure 32 : Coupe en vue de profil d'un second dispositif téléscopique.

Figure 33 : Coupe en vue de profil d'un troisième dispositif téléscopique.

Figure 34 : Coupe en vue de dessus du troisième dispositif téléscopique montrant les roulettes inférieures.

Figure 35 : Vue de profil d'un bras de jonction et d'une chaussure équipée d'une articulation dans sa partie avant.

Figure 36 : Vue de face d'un bras de jonction et d'une chaussure équipée d'une articulation.

Figure 37 : Vue de dessus d'une chaussure, d'un bras de jonction et d'un dispositif téléscopique en coupe.

Figure 38 : Vue en coupe d'une articulation blocable avec un premier mode de déblocage.

Figure 39 : Vue en coupe d'une articulation blocable avec un second mode de déblocage.

Figure 40 : Vue en coupe d'une articulation blocable avec un troisième mode de déblocage.

Figure 41 : Vue latérale en coupe G-G d'une articulation blocable.

Figure 42 : Vue latérale en coupe H-H d'une articulation blocable.

Figure 43 : Vue latérale du rochet cylindrique de blocage de l'articulation inférieure du dispositif téléscopique.

Figure 44 : Vue en coupe du rochet cylindrique.

Figure 45 : Vue latérale en coupe de la pièce de transmission d'efforts à la butée.

Figure 46 : Vue schématique d'ensemble d'un générateur de liquide sous pression comprenant le moteur, le compresseur, les réservoirs et les tiroirs de distribution aux pistons moteurs des dispositifs téléscopiques.

Figure 47 : Vue schématique en coupe des tiroirs de distribution de la figure 46 dans une 2ème position

Figure 48 : Vue schématique en coupe des tiroirs de distribution de la figure 46 dans une 3ème position.

Figure 49 : Vue schématique en coupe des tiroirs de distribution de la figure 46 dans une 4ème position.

Figure 50 : Vue schématique d'ensemble d'un générateur d'air comprimé comprenant le moteur, le compresseur, les réservoirs et les tiroirs de distribution aux pistons moteurs des dispositifs téléscopiques.

Figure 51 : Coupe schématique de profil de l'ensemble de l'appareil selon l'invention à l'exception de la selle, montrant l'emplacement des organes de commande de l'articulation blocable.

Figure 52 : Schéma de principe du système de déblocage hydraulique de l'articulation blocable.

Figure 53 : Schéma de principe d'un premier système de déblocage électrique, par solénoïde, de l'articulation blocable.

Figure 54 : Schéma de principe d'un deuxième système de déblocage, par moteur et vis, de l'articulation blocable.

Figure 55 : Schéma de principe d'une moitié du circuit de commande de l'alimentation-vidange des dispositifs téléscopiques.

Figure 56 : Vue extérieure de profil de l'appareil selon l'invention muni de vérins d'assistance au mouvement pendulaire des jambes.

Figure 57 : Schéma de principe du système d'assistance au mouvement pendulaire des jambes.

Figure 58 : Vue extérieure de profil de l'appareil selon l'invention muni de points d'appuis supplémentaires pour le corps : appui sous les épaules et appui par poignées latérales sur la selle.

Figure 59 : Vue extérieure de dos de la figure 58.

Figure 60 : Vue extérieure de profil de l'appareil selon l'invention avec une première variante sur le dispositif téléscopique.

Figure 61 : Vue extérieur de profil de l'appareil selon l'invention avec une deuxième variante sur le dispositif téléscopique.

Figure 62 : Vue extérieure de profil du bras de jonction muni d'une articulation d'axe vertical a proximité de l'articulation blocable.

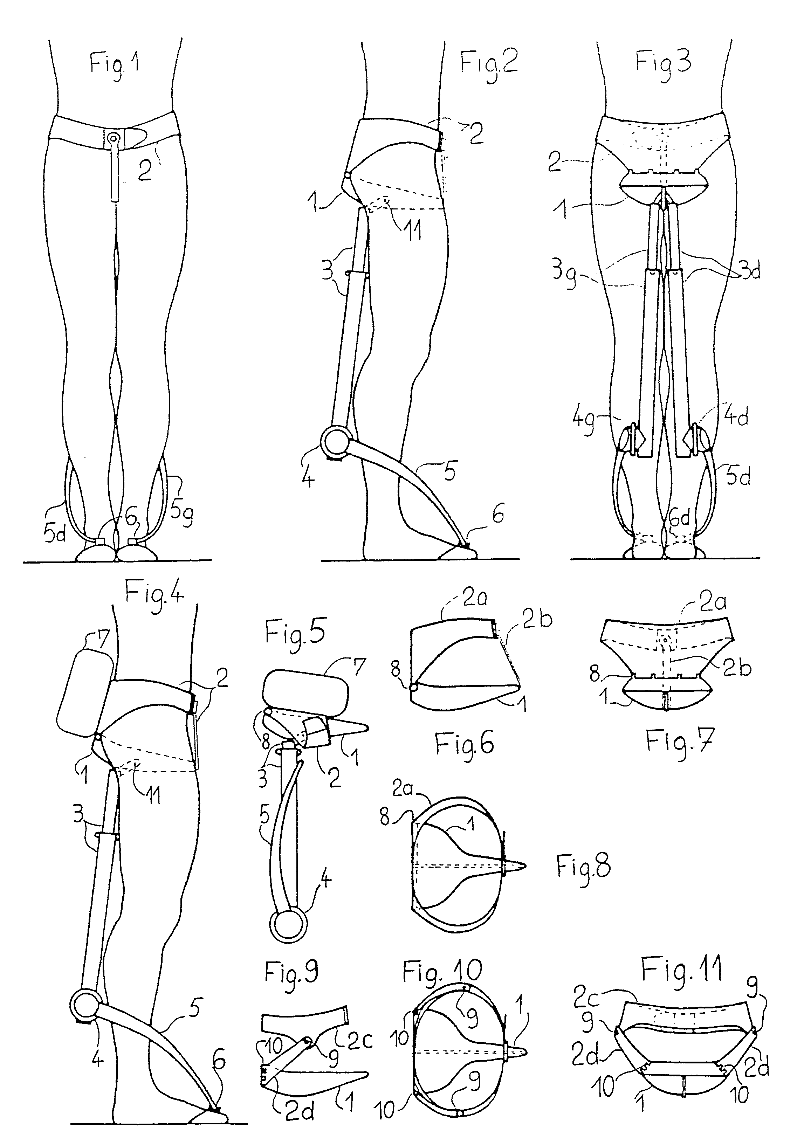

Les figures 1 à 11 représentent l'ensemble de l'appareil selon l'invention dans lequel on distingue :

La selle 1 et son dispositif de fixation aux hanches de l'individu 2. Le dispositif télescopique 3, l'articulation blocable 4, le bras de jonction 5 et l'articulation fixée sur la partie avant de la chaussure 6. Sur les vues autres que de profil les pièces 3, 4, 5, 6 portent la précision gauche g ou droite d.

Sur les figures 1 à 3 on peut voir l'appareil complet représenté sans générateur de fluide comprimé. On voit que les dispositifs télescopiques 3 sont situés derrière les jambes et assez rapprochés l'un de l'autre sans risquer toutefois de se heurter. Cela n'est pas limitatif de l'invention qui comprend aussi les cas où la distance entre les deux articulations blocables 4 serait beaucoup plus importante et permettrait, par exemple de rapprocher cette articulation 4 de l'articulation 6 afin de diminuer la longueur du bras de jonction 5. Dans ce cas, le dispositif téléscopique qui peut être, comme on le verra plus loin curviligne ou rectiligne contournerait partiellement la jambe par l'arrière mais l'ensemble de l'appareil possèderait toujours les mêmes éléments fondamentaux.

Sur la figure 4, l'appareil est représenté avec un générateur 7 de fluide comprimé. Ce dernier est dans ce cas fixé sur le dispositif de fixation aux hanches 2 de la selle mais cela n'est pas limitatif de l'invention. En effet, comme la jonction fonctionnelle avec la selle et le cylindre moteur de chaque téléscope s'effectue par des conduits résistants à la pression mais souples, qui ne sont d'ailleurs pas représentés sur la figure 4, on peut dissocier totalement le générateur de fluide comprimé 7 de la selle ou de ses dépendances. Par exemple, il est donc possible de fixer le générateur 7 sur le dos de l'individu comme un bagage quelconque.

Sur la figure 5, l'appareil est représenté de profil, en position replié pour le transport ou le rangement. On voit que, grâce au relèvement des bras de jonction 5, à l'enfoncement maximal du dispositif téléscopique 3 et au rabattement sur la selle du dispositif de fixation aux hanches 2 supportant le générateur de fluide comprimé 7, l'ensemble du système replié est très peu encombrant et est facile à transporter et à ranger. Dans le cas où le générateur 7 n'est pas fixé sur le dispositif 2 on peut prévoir de la ranger séparément en débranchant par une jonction rapide les deux conduits souples d'alimentation des dispositifs téléscopiques et une prise de courant assurant les liaisons électriques fonctionnelles.

Les figures 6, 7 et 8 représentent un premier type de dispositif de fixation 2 de la selle sur les hanches. Il est composé d'une partie 2a reliée à l'arrière de la selle par une articulation 8 d'axe perpendiculaire au plan médian du corps et de la selle. Le rôle de l'articulation 8 est d'exercer en permanence une poussée vers le haut et vers l'avant sur la selle tout en permettant de légers mouvements angulaires et le repliage sur la selle pour le rangement. Cette partie 2a est de préférence d'une raideur évolutive, afin de maintenir la zône de l'articulation 8 dans une position suffisamment rigide sans nuire au confort de l'individu. La zône située à l'arrière sera donc plus rigide que la zône avant qui se ferme avec une boucle de ceinture. Le matériau composant la partie 2a peut être par exemple un thermoplastique souple tel que le polychlorure de vinyle, soit d'épaisseur évolutive, soit renforcé par endroits par des tissus croisés à 45°. Le matériau peut être aussi un élastomère tel que le polyuréthane par exemple également d'epaisseur évolutive ou renforcé localement par des tissus croisés à 45°.

La partie 2b est une sangle souple, éventuellement faiblement élastique, qui relie l'extrêmité avant de la selle à une liaison démontable sur la boucle de ceinture de la partie 2a.

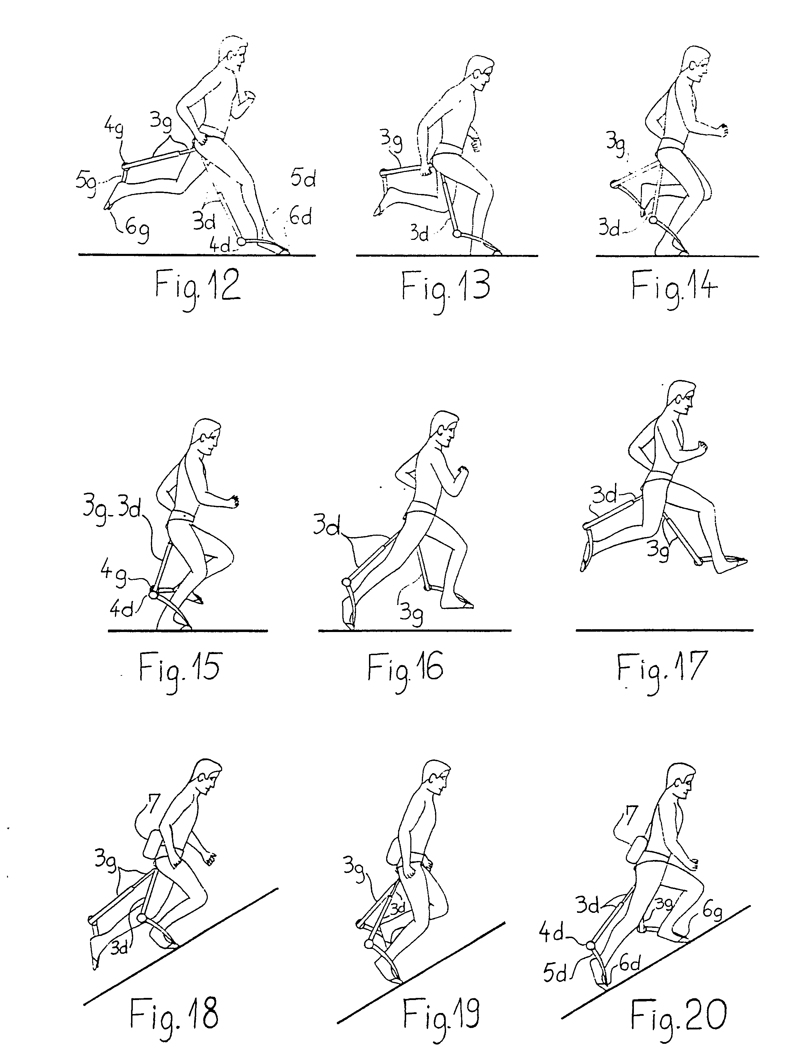

Les figures 9, 10, et 11 représentent un second type de dispositif de fixation 2 de la selle sur les hanches. Il est composé d'une large ceinture 2c munie à sa base, de préférence sur des prolongements inférieurs situés de chaque côté des hanches, de deux articulations 9 qui servent de point d'accrochage à deux bras 2d qui aboutissent à deux articulations 10 solidaires de l'arrière de la selle 1.

La ceinture 2c peut être réalisée dans des matériaux du type utilisé pour le premier dispositif 2 précédemment décrit mais les bras 2d sont en matériau rigide : métal ou plastique rigide tel que polycarbonate par exemple.

L'inclinaison que l'on peut voir sur la figure 11, de l'articulation 10, est destinée à obtenir un repliage peu encombrant des bras 2d contre la selle. L'inclinaison que l'on peut voir sur la figure 9 de l'articulation 10 des bras 2d montre que sur cette vue de profil l'angle que font les bras 2d avec le dessus de la selle 1 est constant. Par conséquent, les points d'accrochage par les articulations 9, qui sont situés au-dessus et au milieu de la selle considérée dans sa longueur, sont suffisants pour assurer le bon maintien en position de la selle pendant le fonctionnement, à fortiori si les articulations des dispositifs téléscopiques sur la selle sont munis du système de stabilisation à trois engrenages qui sera décrit plus loin.

Ces deux dispositifs de fixation de la selle aux hanches 2 peuvent comprendre des variantes et ne sont pas limitatifs de l'invention.

En particulier ils peuvent être adaptés à divers types d'usage de l'appareil ou à des infirmes. On peut notamment compléter l'application de l'effort ascendant sur la selle par la présence de sangles passant sur les épaules et reliées à la selle.

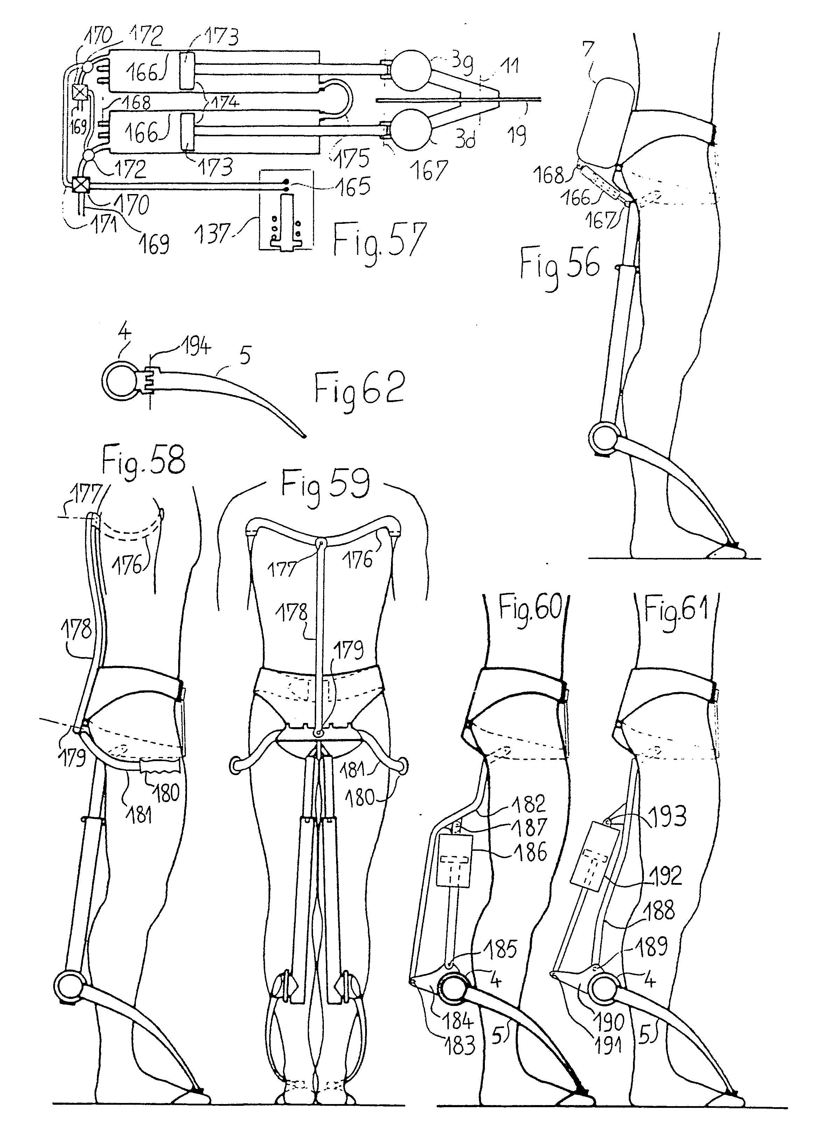

Par ailleurs, comme on le verra plus loin, la selle peut être munie de poignées, représentées sur les figures 58 et 59. Celles-ci peuvent servir de complément d'appui pour le corps lors de l'effort de propulsion exercé par l'appareil et diminuer de ce fait l'effort concentré sous les fesses de l'individu. Mais parallèlement, elles peuvent suffire surtout pour des usages très courts, à maintenir la selle en place avec les mains en l'absence du dispositif de fixation 2 de la selle sur les hanches.

Les figures 12 à 20 font comprendre le mode de fonctionnement global de l'appareil selon l'invention et illustrent la bonne compatibilité géométrique de celui-ci avec le corps humain en déplacéments variés.

En particulier, les figures 12 à 17 représentent plusieurs séquences, chronologiquement disposées, de la course avec bondissement qui est une course normale dans laquelle le fléchissement après atterrissage est un peu plus accentué pour donner un meilleur appel et qui comprend un bond plus long.

La figure 12 est le début d'atterrissage sur jambe droite. Les figures 13 et 14 sont la phase de fléchissement après atterrissage. On peut noter que le fléchissement concerne ici l'articulation de la cheville et celle du genou, tandis que dans la course sans bondissement, également assistable par l'appareil selon l'invention, la répartition des fléchissements sur les articulations naturelles peut être un peu différente. Cependant, dans tous les cas, cette phase de fléchissement correspond à une réduction de la distance séparant l'articulation 6d de l'articulation 11 sous la selle.

La figure 15 représente le moment où le pied gauche dépasse en direction de l'avant le pied droit qui reste en arrière. Ce moment correspond à peu près à la position de fléchissement maximum. C'est par ailleurs aussi le moment où le dispositif téléscopique de droite 3d qui reste en arrière est croisé par le dispositif téléscopique de gauche 3g qui va vers l'avant.

Les efforts accomplis par le système musculaire de l'individu entre la position représentée sur la figure 12 et celle de la figure 15 sont des efforts résistants ou passifs. Ils correspondent à une fatigue inutile à la propulsion mais ils sont nécessaires pour placer le corps dans une position favorable, ou position d'appel, au début de la phase propulsive.

C'est pourquoi l'une des fonctions de l'appareil selon l'invention est de récupérer l'énergie mise en jeu à ce moment afin de la restituer lors de la phase propulsive. Pour cela, l'individu doit se laisser peser sur la selle de l'appareil en n'exerçant durant cette phase que le minimum d'efforts musculaires nécessaires à son équilibre. L'appareil stocke l'énergie soit par compression de son ressort de propulsion, soit par compression d'une vessie renfermant un gaz comme on le verra plus loin. Dans les deux cas, la fonction d'amortissement de l'atterrissage après le bond est bien respectée également, grâce à l'élasticité des procédés de stockage.

Entre les figures 15 et 16 a lieu la propulsion musculaire et, parallèlement à celle-ci, la propulsion effectuée par l'appareil selon l'invention puisque, comme on le voit, la distance entre l'articulation 6d et l'articulation des dispositifs téléscopiques sur la selle s'accroît continuellement pendant cette période. Il suffit donc que l'effort moteur d'extension du dispositif téléscopique s'applique pendant cette phase. Si cet effort d'extension est dû à la détente du ressort de propulsion préalablement comprimé dans la phase comprise entre la figure 12 et la figure 15, aucun signal n'est nécessaire pour déclencher cet effort. Si cet effort d'extension est dû à la poussée d'un fluide sur un piston contenu dans le dispositif téléscopique 3d, le dispositif de pilotage décrit plus loin fait débuter l'effort au voisinage de la position décrite sur la figure 15 et le fait cesser au voisinage de la position décrite sur la figure 16.

On peut résumer toutes ces actions mécaniques et musculaires décrites entre les figures 12 et 16 en disant que l'appareil consacre à aider la phase propulsive, au minimum l'énergie qu'il a prélevée sur le travail musculaire résistant ou passif pendant la phase d'atterrissage et éventuellement, ajoute une énergie complémentaire provenant d'une source de fluide comprimé. Mais dans ces deux cas, l'individu reste libre de consacrer à la phase propulsive l'effort musculaire de son choix, ce dernier pouvant être plus intense qu'en temps normal car il n'est pas tenu d'exercer en plus l'effort musculaire d'atterrissage. On observera que l'opération d'assistance à la propulsion effectuée par l'appareil selon l'invention n'entraîne pas de contraintes supplémentaires sur le squelette des jambes de l'individu et sa fragile articulation sur le bassin. L'effort s'exerce directement à la base de la colonne vertébrale dont l'usage des déplacements à cheval a démontré depuis longtemps l'aptitude à recevoir des efforts importants : lorsqu'un cheval est au trot, son cavalier reçoit en permanence des efforts capables de la faire décoller de sa selle. Ces efforts sont l'ordre de grandeur maximum de ceux que transmet l'appareil sous la selle de l'individu mais le système de commande permet d'obtenir à volonté toute la gamme des efforts intermédiaires. Par ailleurs, comme on le verra à la fin, divers systèmes annexes permettent à volonté de répartir l'effort propulsif entre la base et le sommet de la colonne vertébrale.

Sur les figures 12 à 16, l'articulation blocable droite 4d se trouve, pour le dispositif de la jambe droite, en position bloquée par son rochet.

En revanche, cette articulation 4d se débloque dès la fin de l'opération représentée figure 16, de sorte que sur la figure 17 elle est débloquée. On peut continuer les opérations chronologiques sur cette articulation 4d de la jambe droite en passant de la figure 17 à la figure 12 et en s'intéressant à l'articulation 4g du dispositif de la jambe gauche. Sur la figure 12, l'articulation 4g est débloquée mais l'angle du bras de jonction gauche 5g avec le dispositif téléscopique gauche 3g a encore peu varié par rapport à l'angle représenté sur la figure 17 entre le bras de jonction 5d et le dispositif téléscopique 3d du côté droit. Mais dès l'atterrissage, l'individu doit continuer à avancer la cuisse gauche vers l'avant tout en maintenant le pied gauche à une position suffisamment élevée pour ne pas risquer de buter sur un obstacle sur le sol.

L'appareil selon l'invention ne doit exercer aucun effort s'opposant à ce mouvement naturel sous peine de rendre le déplacement de l'individu désagréable et dangereux. Ce but est atteint grâce au déblocage de l'articulation 4g, à la liberté de mouvement dans l'articulation 6g et à la très faible inertie de la partie avant du bras de jonction 5g. En effet, en supposant que le dispositif téléscopique 3g ait une course de remontée suffisante pour permettre, sans débloquer l'articulation 4g, l'avance du pied gauche vers l'avant sans heurter le sol, toute la masse de la moitié inférieure du dispositif téléscopique et toute la masse du bras de jonction 5g seraient constamment reliées au pied par l'articulation 6g. Cela constituerait une inertie élevée, gênante et dangereuse pour l'équilibre. Grâce à l'articulation déblocable 4g, l'inertie du dispositif téléscopique 3g n'est ressentie par le pied que pendant le mouvement pendulaire vers l'avant autour de l'articulation de la selle. Or, d'une part ce mouvement pendulaire est beaucoup moins rapide que le fléchissement du genou gauche entre les figures 13 et 14, d'autre part la masse du dispositif 3g favorise ce mouvement puisqu'il a lieu vers le sol au contraire du précédent.

Le rôle essentiel de l'articulation 4 est donc de faire en sorte que les influences inertielles du dispositif sur l'articulation 6 du pied et les mouvements de remontée de la jambe soient très faibles. Par ailleurs, comme on le verra plus loin, le déblocage de l'articulation 4 permet de disposer d'un temps relativement long pour vider le cylindre moteur du dispositif téléscopique 3 et de récupérer la plus grande partie de l'énergie de détente du gaz comprimé qui peut s'y trouver. Enfin, grâce au déblocage de l'articulation 4, l'appareil est peu encombrant pour le rangement.

A partir de la figure 15, l'angle, alors minimum, formé par le dispositif téléscopique 3g et le bras de jonction 5g de la jambe gauche, augmente et atteint son maximum peu de temps avant l'atterrissage du pied gauche après le bond représenté figure 17 qui ramène à la figure 12 en inversant la jambe et ainsi de suite.

L'articulation 4 est munie d'un système de blocage à rochet qui permet d'augmenter l'angle formé par le dispositif téléscopique 3 et la bras de jonction 5 mais qui bloque l'articulation dans le cas inverse, quel que soit l'angle atteint. Le système de déblocage de l'articulation 4 consiste à neutraliser le blocage à rochet pour permettre quand même la diminution de l'angle formé par 3 et 5. Il faut donc que le système de déblocage de l'articulation 4g cesse son action sur le rochet dès que le dispositif téléscopique concerné, le gauche en l'occurence, dépasse en allant vers l'avant le système téléscopique 3d de l'autre jambe. Ainsi, quelle que soit l'augmentation de l'angle entre 3g et 5g qui aura été causée par l'action du pied gauche après ce moment et avant qu'il touche le sol, le mouvement de diminution de cet angle sera alors impossible. Les moyens techniques pour y parvenir seront décrits plus loin.

Les figures 18 à 20 représent un autre mode de fonctionnement de l'appareil qui correspond à la montée d'une pente en l'absence de tout mouvement de bondissement. C'est donc le mode d'utilisation le plus éloigné de celui qui est décrit sur les figures 12 à 17 mais il est aussi possible de gravir des pentes en mode bondissant, c'est-à-dire intermédiaire entre les deux modes décrits.

En l'absence de bondissement, la seule assistance possible à la propulsion, compatible avec une longue autonomie, est une pression de fluide s'exerçant sur un piston contenu dans les dispositifs téléscopiques 3. L'individu est donc - schématiquement représenté sur les vues 18 à 20 avec un générateur de fluide comprimé 7. Comme il a été dit plus haut, ce générateur 7 peut petre accroché en divers points de l'individu. Cependant la présence d'une boîte extérieure contenant le générateur 7 n'est pas obligatoire sauf dans le cas d'un groupe motocompresseur et ce dernier n'est pas limitatif de l'invention.

Il est en effet possible d'envisager, dans le cadre de l'invention, un principe de combustion à l'intérieur même des cylindres contenus dans les dispositifs téléscopiques. Cette combustion peut concerner un mélange carburant + air fonctionnant selon un cycle à 4 temps que l'on peut imaginer en observant sur les figures 12 à 17 les variations de longueurs des dispositifs téléscopiques 3 et en effectuant une analogie avec un cycle de Beau de Rochas ou Diesel. Pour appliquer cette combustion au type d'utilisation décrit sur les figures 18 à 20, il faudrait toutefois envisager un cycle à deux temps ce qui semble plus difficile.

Cette combustion peut aussi être, pour tous les cas d'utilisation, celle d'un composé chimique pouvant brûler à l'abri de l'air et générer une forte pression gazeuse. Il suffit alors d'introduire automatiquement et d'allumer une dose unitaire à l'intérieur de la cavité étanche expansible d'un dispositif téléscopique au début de chaque phase propulsive et de prévoir un échappement des gaz en fin de course d'expansion. Ces composés chimiques peuvent être des poudres apparentées à celles des fusées, avec une combustion relativement lente.

Tous ces procédés éventuellement envisageables auraient cependant de très mauvais rendements énergétiques, donc les groups moto-compresseurs sont nettement plus économiques à l'usage et offrent aussi des conditions d'emploi beaucoup plus souples.

On verra à la fin, au chapitre des variantes de l'invention, que si on se limite à de très courtes autonomies de l'appareil, d'autres sources d'énergie à bon rendement sont possibles et ne nécessitent pas non plus de boîtier 7.

Sur la figure 18, l'individu vient de porter le poids de son corps sur son pied droit. A ce même moment, la pression fluide commence à s'exercer sur le piston du système téléscopique droit 3d. Cela tend à augmenter la distance entre l'articulation 6d du pied droit et l'articulation de la selle, donc à aider la propulsion de l'individu dans la côte.

Sur la figure 19, la propulsion par la jambe droite aidée par le dispositif téléscopique droit 3d est en cours. La jambe gauche de l'individu monte parallèlement à la pente grâce au déblocage de l'articulation 4g et, dans une proportion variable, grâce au raccourcissement progressif du dispositif téléscopique 3g de la jambe gauche qui est en cours de vidange.

Sur la figure 20 la propulsion par la jambe droite est pratiquement terminée et le pied gauche est déjà en contact avec le sol. L'angle entre le dispositif téléscopique gauche 3g et le bras de jonction gauche 5g a en général repris la valeur qu'il avait sur la figure 18. Cependant il se peut que dans une pente très forte, montée lentement sans bondir ou avec une foulée trop longue vers l'avant, ou en présence d'un obstacle momentané rencontré par le pied gauche, cet angle ne puisse pas reprendre sa valeur de la figure 18. Dans ces cas exceptionnels, le rochet de l'articulation 4 interdit le relèvement du bras 5g quel que soit l'angle et la poussée due à l'expansion téléscopique peut avoir lieu quand même. L'individu doit simplement terminer la foulée propulsive de la jambe gauche par sa seule force musculaire.

La course motrice des dispositifs téléscopiques qui est, dans le cas représenté, à titre indicatif et nullement limitatif de l'invention, de l'ordre de 25 à 30 cm pour un homme de 1,80 m permet par exemple de gravir sans problème des escaliers normaux à marches de 18 cm de hauteur.

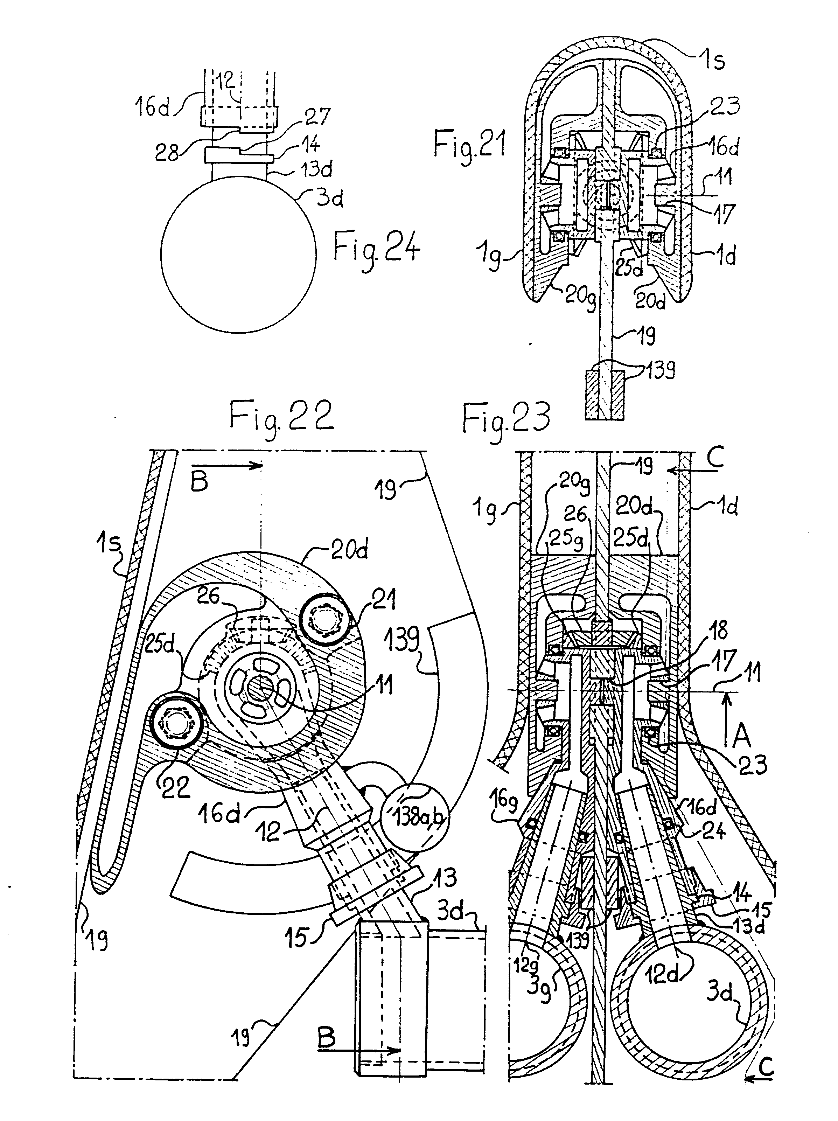

Les figures 21 à 24 décrivent un premier mode de réalisation des articulations situées en haut des dispositifs téléscopiques 3 et sur la selle 1. On distingue d'une part l'articulation 11 d'axe perpendiculaire au plan médian de la selle et du corps, qui relie les dispositifs téléscopiques 3 à la selle 1, et d'autre part les articulations d'axe 12, sensiblement parallèles au plan médian de la selle. Ces articulations 12 permettent d'écarter les dispositifs téléscopiques 3 du plan médian du corps ce qui donne à l'utilisateur la possibilité d'écarter latéralement ses pieds. Par contre, les articulations 12 sont munies d'un système de butée en rotation qui interdit à la base des dispositifs téléscopiques 3 de toucher le plan médian afin que les deux dispositifs 3g et 3d ne puissent jamais se heurter. Notons que la présence des articulations 12 n'est pas limitative de l'invention puisque l'appareil peut remplir son rôle en leur absence mais avec moins de confort pour l'individu.

Les figures 21 à 24 décrivent un système d'articulations 11 et 12 qui permet également l'alimentation en fluide comprimé des systèmes téléscopiques 3 mais le principe reste évidemment valable dans le cas où il n'est pas nécessaire d'alimenter en fluide comprimé.

La description concerne uniquement le côté droit de l'appareil puisque le côté gauche est identique.

On distingue sur les figures 22 et 23 le haut de téléscope 3d auquel est soudé un axe creux 13 porteur d'un épaulement 14 qui retient un écrou 15. L'axe creux 13 qui est l'axe de l'articulation 12 peut tourner librement dans un alesage de la pièce de raccordement 16 qui comporte à son autre extrémité un alésage 17 et un tronçon d'axe plein 18 qui constituent la moitié de l'articulation 11 pour le côté droit. L'axe creux 13 est immobilisé axialement dans la pièce 16 grâce à son écrou 15 qui est vissé sur celle-ci.

Le tronçon d'axe plein 18 tourne librement dans un alésage pratiqué dans une surépaisseur de la cloison médiane 19 de la selle 1. Les parois latérales de la selle 1 sont repérées 1g et 1d sur les figures 21 et 23 et le sommet de la selle 1 est repéré 1s sur les figures 21 et 22.

L'alésage 17 de la pièce de raccordement 16 reçoit un tronçon d'axe qui est solidaire d'une pièce 20d qui est fixée par les boulons 21 et 22 sur la pièce 20 g en enserrant la cloison centrale 19.

Les pièces 16 et 20 comportent des évidements intérieurs pour le passage des fluides et les articulations 11 et 12 possèdent respectivement les joints d'étanchéité 23 et 24. La pièce 20 s'éloigne rapidement du plan médian en allant vers l'arrière de la selle où se font les branchements de canalisations d'alimentation, afin de libérer l'espace sous la selle dans la zône de débattement pendulaire des sommets des dispositifs téléscopiques 3.

Les pièces 16g et 16d sont munies d'un secteur de couronne dentée cônique 25g et 25d centré sur l'axe de l'articulation 11, qui coopèrent avec une roue dentée cônique 26 qui tourne librement sur un axe soudé sur la cloison médiane 19. De cette façon, l'axe de cette roue dentée et la selle dont il est solidaire conserve le même angle avec la bissectrice de l'angle formé par les pièces de raccordement 16d et 16g donc avec les dispositifs téléscopiques 3d et 3g.

Or, on voit sur les figures 12 à 20 que l'angle formé par la bissectrice de l'angle des deux dispositifs téléscopiques 3 avec le tronc varie assez peu dans l'ensemble des cas. Par conséquent, les secteurs dentés 25d et 25g et la roue cônique 26 maintiennent le sommet 1s de la selle dans une direction favorable en permanence. L'usage de ces secteurs 25 et roue dentée 26 n'est ni obligatoire ni limitatif de l'invention mais il présente, entre autres, l'avantage de pouvoir si besoin est, positionner l'axe de l'articulation 11 à une distance notable en arrière ou en avant du pont de la selle qui se trouve sous le centre résultant des poussées verticales que le corps exerce sur celle-ci. Ainsi, par exemple, il serait possible, dans le cas des figures 22 et 23 de placer l'articulation 11 beaucoup plus près de l'axe longitudinal des dispositifs téléscopiques 3, sans que la selle s'affaisse vers l'avant sous le poids du corps.

La figure 24 qui décrit le principe de la butée sur l'articulation d'axe 12 montre le redan 27 pratiqué dans l'épaulement 14 de l'axe creux 13d. Ce redan coopère avec un redan 28 de la pièce de raccordement 16d lorsque l'axe creux 13d est enfoncé à fond dans la pièce 16d et est maintenu axialement par l'écrou 15 non représenté.

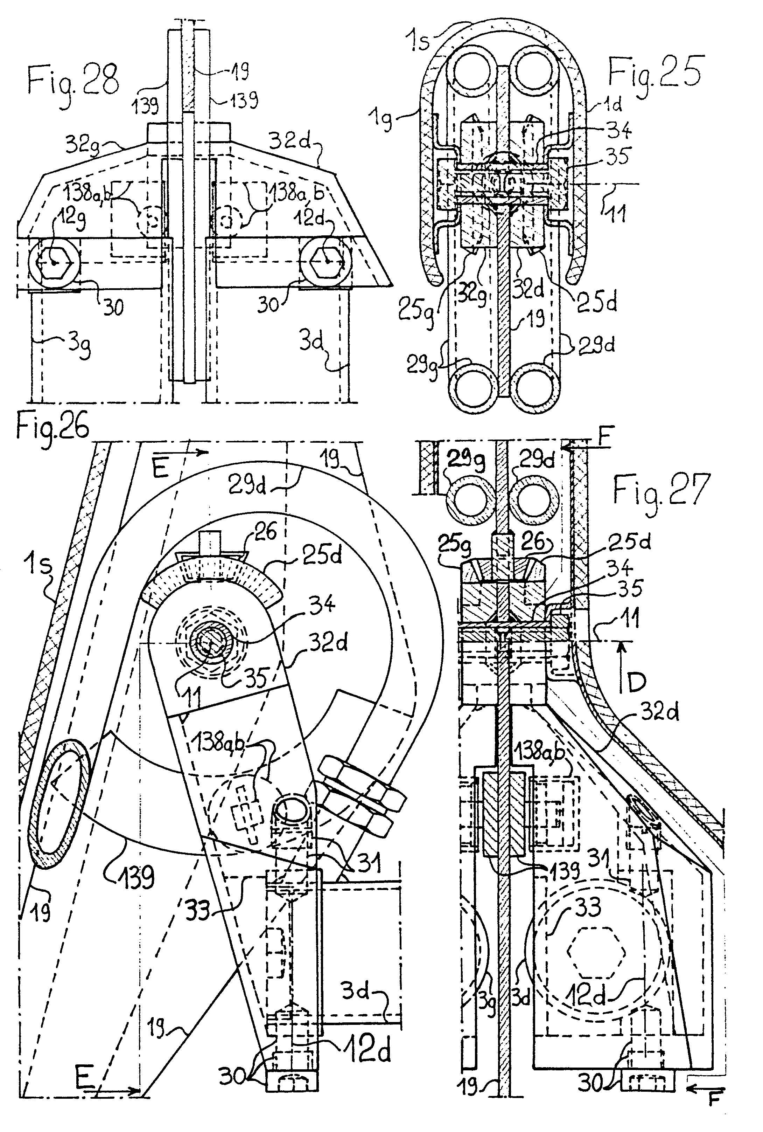

Les figures 25 à 28 décrivent un second mode de réalisation des articulations situées en haut des dispositifs téléscopiques 3 et sur la selle 1. Sur ces figures on voit que les alimentations en fluide des dispositifs téléscopiques 3d et 3g sont assurées, indépendamment des articulations 11 et 12 par des canalisations souples 29g et 29d. Les articulations 12g et 12d sont situées au sommet des dispositifs téléscopiques 3d et 3g, leur axe étant le plus éloigné possible du plan médian. Elles consistent en deux vis 30 et 31 dont l'extrêmité est un axe non fileté. Ces vis 30 et 31 sont vissées dans la pièce 32d de raccordement entre les articulations 11 et 12 et leur extrêmité non filetée traverse une longueur non filetée de la pièce 32d et s'enfonce dans un alésage solidaire du dispositif téléscopique 3d. La butée de cette articulation 12, interdisant au dispositif téléscopique 3d de dépasser le plan médian de la selle, est une cloison 33 de la pièce de raccordement 32d au-dessous de laquelle vient appuyer le sommet du dispositif 3d.

L'articulation 11 est constituée par un tube creux 34 soudé sur la cloison médiane 19 dont l'extérieur tourne librement dans un alésage de la pièce de raccordement 32d. L'intérieur du tube 34 est fileté et reçoit les vis 35 d'immobilisation axiale.

Enfin, ce deuxième mode de réalisation peut également être muni des deux secteurs 25 et de la roue dentée 26 décrits pour le premier mode.

Les deux modes de réalisation de l'articulation sur la selle et des articulations sur le haut des dispositifs téléscopiques qui ont été décrits ne sont pas limitatifs de l'invention ; on peut en particulier combiner leurs aspects techniques entre eux d'autres manières ou au contraire dissocier davantage les fonctions diverses. Par ailleurs, l'axe de l'articulation 11 a été supposé rectiligne et commun à l'articulation des dispositifs téléscopiques gauche et droit. Mais on peut aussi imaginer que l'axe 11 ait une partie droite et une partie gauche n'étant pas tout à fait perpendiculaires au plan médian de la selle pour diverses raisons, tout en restant symètriques par rapport à ce plan. Cette disposition pouvant très éventuellement constituer un choix technique. La particularité fondamentale de l'axe 11 sera donc pour l'ensemble des cas d'être sensiblement perpendiculaire au plan médian de la selle et du corps.

Les figures 22, 23, 26, 27 et 28 décrivent aussi des pièces nécessaires au pilotage qui seront examinées plus loin.

La selle 1 a une structure résistante qui s'organise ici autour de la robuste cloison médiane 19 mais d'autres dispositions sont envisageables à condition qu'elles permettent au sommet des dispositifs téléscopiques 3 de remonter en fin de mouvement pendulaire autour de l'articulation 11, le plus haut possible donc très près de la surface 1s de la selle.

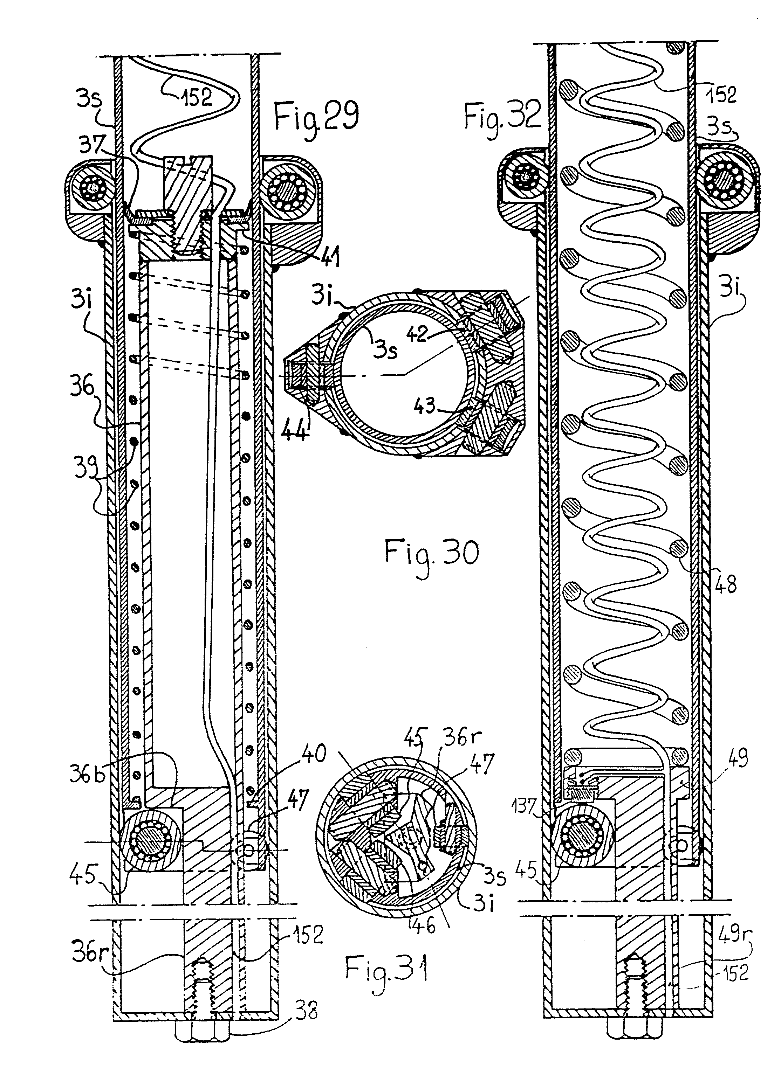

Les figures 29 à 34 décrivent les dispositifs téléscopiques 3 dans différents contextes.

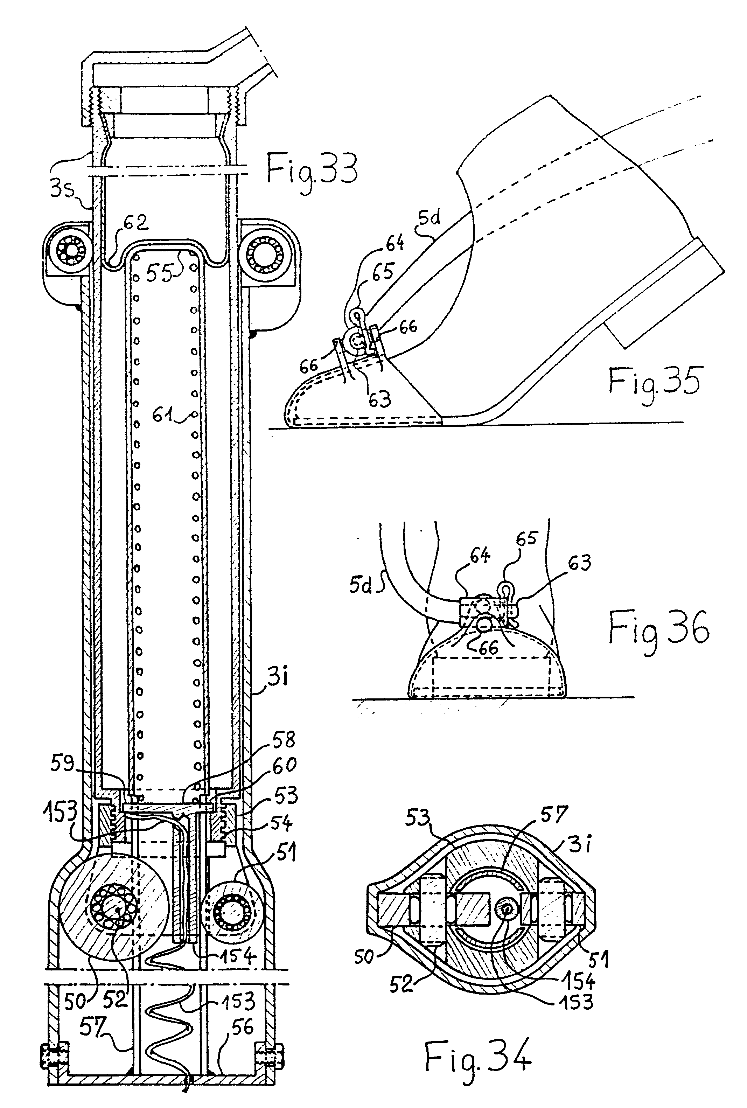

Le dispositif téléscopique est, dans tous les cas décrits sur les figures 29 à 34, composé de deux tubes concentriques mais une section cylindrique n'est en aucun cas limitative de l'invention : on peut utiliser comme sur la figure 33, partie inférieure, des tubes de section particulière, carrés, rectangulaires, etc ... ; on peut aussi utiliser des profilés emboîtés et coulissants dont le périmètre n'est pas fermé. La notion de téléscope désigne toutes les formes qui peuvent s'emboîter l'une dans l'autre et coulisser axialement. De plus, comme il est dit plus haut, l'axe du téléscope n'est pas obligatoirement rectiligne : on peut faire appel à un téléscope curviligne caractérisé par un rayon de courbure constant et identique pour les deux pièces emboîtées. la notion de dispositif téléscopique désigne le téléscope et ses annexes, permettant, d'une part le coulissement en présence d'un couple, d'autre part, l'application d'une force d'extension par un ressort ou un fluide, enfin les ressorts de rappel éventuels.

Par ailleurs, les dispositifs décrits dans les figures 29 à 34 présentent toujours leur tube femelle extérieur en bas. Cette disposition est préférable à la disposition inverse car le tube mâle étant plus fragile est moins vulnérable au voisinage de la selle et de plus, il est plus facile de fixer l'articulation blocable 4 sur le tube femelle que sur le tube mâle. Cependant, cette disposition n'est pas limitative de l'invention qui comprend par conséquent aussi le cas inverse

Les figures 29 à 31 sont relatives à un premier dispositif téléscopique, dont le mode d'exercice de la force d'extension est dû à l'action d'un fluide comprimé sur un piston. Si la qualité de l'étanchéité du piston est très bonne, le fluide pourra être un liquide ou un gaz, si elle est juste passable, le fluide ne pourra être que de l'air, pour lequel les fuites n'ont que peu d'importance.

Dans le cas des figures 29 à 31 le cylindre intérieur dans lequel coulisse le piston 36 muni de la rondelle d'étanchéité plastique 37 est la paroi interne de la partie supérieure 3s du dispositif téléscopique. Il pourrait aussi exister une pièce cylindrique supplémentaire concentrique à la pièce 3s pour accueillir un piston de petit diamètre fonctionnant à plus haute pression.

La partie inférieure 36b du piston sert de butée pour limiter l'allongement maximum. Le piston 36 est prolongé par une rallonge 36r, qui est fixée par une vis 38 à la base de la partie inférieure ou femelle 3i du dispositif téléscopique 3.

Un ressort de rappel 39 qui tend à réemboîter les parties 3s et 3i du téléscope en l'absence de forces supérieures à la sienne prend appui sur une collerette 40 située vers l'extrêmité inférieure de la partie 3s du téléscope, et exerce son action sur une collerette 41 située à la partie supérieure du piston 36.

Le coulissement des parties supérieure mâle 3s et inférieure femelle 3i en présence d'un couple est assuré par deux groupes de roulettes sur roulements à aiguilles ou à rouleaux.

Le groupe supérieur de roulettes, dont les axes sont solidaires de la partie inférieure femelle 3i du dispositif téléscopique est fixé à l'extrêmité supérieure de cette pièce 3i et comprend trois roulettes visibles en coupe en vue axiale de dessus sur la figure 30.

On distingue deux roulettes principales 42 et 43 dont les axes sont perpendiculaires à l'axe du téléscope et inclinés d'environ 60° par rapport au plan médian du corps, la surface de contact des roulettes étant tangente à la partie mâle ou supérieure 3s du téléscope. On distingue aussi une roulette secondaire 44 dont l'axe est perpendiculaire à l'axe du téléscope et sensiblement perpendiculaire au plan médian du corps. Ce système de 3 roulettes maintient les tubes 3s et 3i éloignés l'un de l'autre même en présence d'un effort radial important. L'effort principal de couple est reçu par les roulettes 42 et 43 qui sont disposées à l'avant du téléscope par rapport à l'individu.

Le groupe inférieur de roulettes dont les axes sont solidaires de la partie supérieure ou mâle 3s du dispositif téléscopique est fixé à l'extrêmité inférieure de la pièce 3s et comprend trois roulettes visibles en coupe en vue de dessus sur la figure 31.

On distingue là encore deux roulettes principales 45 et 46 et une secondaire 47 dont la disposition des axes est symétrique de celle des roulettes supérieures 42 à 44. Les roulettes principales, qui reçoivent l'effort de couple, sont alors disposées à l'arrière du dispositif téléscopique 3 par rapport à l'individu et roulent sur la partie interne du tube femelle ou inférieur 3i du téléscope.

La section droite de la rallonge 36r du piston 36 est visible sur la figure 31 et on constate qu'elle permet une rotation notable du tube inférieur 3i du téléscope dont elle solidaire, par rapport au tube supérieur 3s dont sont solidaires les roulettes 45 à 47 sans qu'elle entre en contact avec ces roulettes. Cela donne un degré de liberté supplémentaire à ce type de dispositif téléscopique, qui est une rotation limitée, autour de son axe. Il en résulte, pour l'individu, la possibilité d'effectuer une rotation à axe vertical avec son pied.

Compte-tenu des efforts de couple élevés que subissent les dispositifs téléscopiques 3, il est important que les tubes 3s et 3i soient réalisés, ainsi que les roulettes, en acier à très hautes performances mécaniques, ce qui leur confère une longue durée de vie en service et une masse minimale.

D'autres éléments visibles sur les figures 29 et 31 seront décrits au chapitre du pilotage.

Le sommet de la pièce 3s n'est pas représenté mais il peut être, de façon non limitative, de l'un des types représentés sur les figures 22 à 28.

La figure 32 représente un dispositif téléscopique 3 dont les tubes 3s et 3i ainsi que les six roulettes de coulissement sont identiques à ceux décrits sur les figures 29 à 31.

Son actionnement mécanique est dû à un puissant ressort spirale 48 qui appuie sur la tête d'un piston 49 prolongé par une rallonge 49r semblable à la rallonge 36r du cas précédent. L'autre extrêmité du ressort 48 prend appui sous le couvercle de la partie supérieure 3s du dispositif téléscopique où peut se trouver un système de réglage de la précontrainte composé d'une vis et d'un écrou. Il n'y a pas de ressort de rappel puisque le ressort 48 est précontraint ce qui, en l'absence de forces extérieures au téléscope, ramène l'extrêmité inférieure du piston 49 au contact d'une butée solidaire de l'extrêmité inférieure du tube mâle ou supérieur 3s du téléscope. Cette butée peut être une des roulettes 45 ou 46. La précontrainte du ressort 48 peut éventuellement être très faible ou nulle.

Dans le cas de la figure 32, le relèvement du pied après la phase propulsive n'est don dû qu'au mouvement angulaire vers le haut du bras de jonction 5 grâce au déblocage de l'articulation 4.

Toutefois, il existe d'autres moyens pour mettre en jeu la force du ressort 48, permettant notamment de raccourcir le téléscope 3 après le phase propulsive. La disposition décrite sur la figure 32 pour le ressort 48 n'est donc pas limitative de l'invention.

Le dispositif téléscopique représenté sur les figures 33 et 34 possède un groupe de trois roulettes supérieures identique à celui décrit sur les figures 29 et 30.

Son guidage inférieur ne comprend en revanche que deux roulettes 50 et 51 dont la plus grosse, placée à l'arrière du téléscope, reçoit les efforts de couple. Ces roulettes roulent sur l'intérieur de la partie femelle ou inférieure 3i du téléscope dont la section visible sur la figure 34 est allongée sensiblement parallèlement au plan médian. Cela permet d'utiliser une roulette 50 de diamètre relativement important. L'axe 52 de celle-ci n'est plus directement solidaire de l'extrêmité inférieure du tube 3s mais il est fixé dans une pièce 53 qui est reliée à la pièce 3s par un filetage à filets carrés 54. Le filetage n'étant pas en butée, la pièce 53 peut tourner librement par rapport à la pièce 3s. Le filet carré n'est pas limitatif de l'invention, son but étant simplement de tourner plus facilement qu'un filet triangulaire.

Cette disposition décrite est commode pour la transmission des efforts exercés sur la tête de piston 55 jusqu'au fond 56 de la partie inférieure 3i du téléscope. En effet, la rallonge inférieure 57 du piston 55 est alors un tube ou une tige fendue diamètralement pour laisser avec un faible jeu le passage des roulettes 50 et 51. Lors de la rotation de la partie inférieure 3i du téléscope par rapport à la partie supérieure 3s, le tube fendu 57 et les roulettes 50 et 51 sont donc entraînées simultanément, contrairement aux cas des roulettes 45, 46 et 47 et de la rallonge 36r ou 49r.

Une pièce 58 coulisse également à travers les fentes du tube 57 et ses deux protubérances 59 et 60 vont prendre appui sur un alésage avec épaulement pratiqué dans l'extrêmité inférieure de la pièce 3s.

Ainsi la pièce 58 est solidaire du tube 57 en rotation et solidaire de la pièce 3s en translation. Elle sert d'une part d'appui au ressort de rappel 61 du dispositif téléscopique et d'autre part au passage des fils de pilotage comme on le verra plus loin.

Le mode d'exercice de la force extensive du dispositif téléscopique de la figure 33 est une membrane cylindrique déroulable concentriquement 62 fixée de façon étanche au sommet du tube 3s comme on le voit sur la figure 33. L'avantage de ce procédé est l'absence totale de fuite et il convient particulièrement bien pour l'usage des liquides sous pression. La membrane 62 est en élastomère, de préférence armé de fibres organiques ou minérales, de préférence sous forme d'un tissu cylindrique dont les fils constituant les génératrices sont la majorité et les fils des directrices circulaires sont en minorité ou sont en élastomère. De cette façon, la membrane 62 résiste à de forts efforts axiaux et peut subir facilement un roulement concentrique concernant deux diamètres notablement différents.

Les dispositifs téléscopiques des figures 29 à 34 sont tous représentés en position d'extension maximale, de sorte que leur ressort de rappel 39 ou 61 est comprimé au maximum et que la distance entre la groupe de roulettes supérieures et le groupe inférieur est minimale.

Comme il est dit plus haut, les systèmes de guidage décrits permettant le coulissement des divers éléments des téléscopes entre eux ainsi que les moyens décrits pour exercer une action mécanique d'extension, par un ressort ou par un fluide comprimé et les moyens décrits pour permettre une rotation axiale des téléscopes ne sont pas limitatifs de l'invention. Les systèmes décrits peuvent d'une part être combinés différemment entre eux. Il peuvent d'autre part être complétés par :

-des téléscopes comprenant plus de deux éléments emboîtés et coulissants,

-des systèmes de guidage comportant plus ou moins de roulettes qu'il est indiqué,

-des systèmes de guidage à circulation de billes ou à billes guidées par cages intermédiaires,

-des systèmes de guidage remplaçant les roulettes ou billes faiblement chargées par des pièces de frottement, etc ...

-des dispositions internes différentes pour les pistons devant transmettre une pression fluide et comprenant par exemple des soupapes et réservoirs internes aux téléscopes pour limiter les transferts de fluides dans certaines configurations, etc ...

-des mises en oeuvre différentes pour le ressort devant effectuer le stockage et la restitution d'énergie, etc ...

Les figures 35 et 36 représentent un mode, également non limitatif de réalisation de l'articulation 6 sur la partie avant de la chaussure.

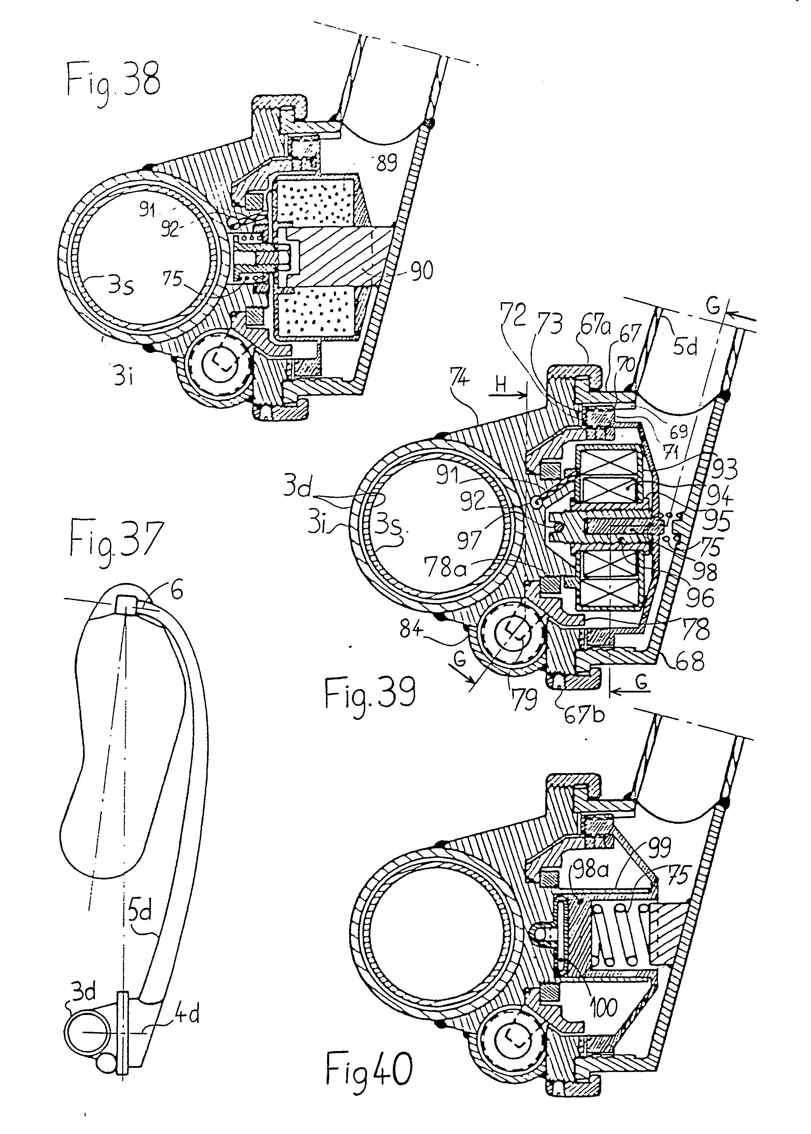

Si l'on considère le pied en vue de dessus comme indiqué sur la figure 37, le but recherché dans l'articulation 6 est d'autoriser de grands mouvements angulaires autour d'un axe sensiblement perpendiculaire au plan médian du corps et d'interdire les mouvements de rotation à axe vertical qui pourraient conduire à heurter le bras 5d avec la cheville ou la chaussure ou le bas du mollet. De plus, et en fonction des usages les plus courants réservés à chaque type d'appareil selon l'invention, il est parfois utile que l'articulation autorise un mouvement de roulis du pied, d'amplitude limitée. Ce mouvement s'effectue autour d'un axe sensiblement longitudinal du pied.

Le mouvement de rotation du pied autour d'un axe horizontal sensiblement perpendiculaire au plan médian du corps, donc sensiblement perpendiculaire à l'axe longitudinal du pied et sensiblement parallèle au sol, est obtenu grâce à l'axe 63, prolongeant le bras de jonction 5, qui peut tourner librement dans un alésage d'une pièce cylindrique 64. Le montage et le démontage de l'axe 63 dans la pièce 64 se font de façon immédiate en soulevant ou en enfonçant dans un trou de la pièce 64 la goupille 65 qui immobilise axialement l'axe 63 grâce à une gorge circulaire de celui-ci. On doit préciser ici que pour s'équiper de l'appareil, l'individu commence par mettre des chaussures équipées de la pièce 64. Puis il pose verticalement sur le sol l'appareil, qui prend appui sur la base des dispositifs téléscopiques 3 et les articulations 4. Il s'assoit alors sur la selle et boucle la ceinture d'accrochage de celle-ci. Enfin, et toujours dans la position assise, il enfonce les axes 63 des bras de jonction 5 dans chacune des pièces 64 et enfonce les goupilles 65.

La pièce cylindrique 64 possède deux tronçons d'arbre latéraux 66 qui matérialisent un axe de rotation perpendiculaire à l'alésage de la pièce 64 et sensiblement longitudinal par rapport au pied. Ces deux tronçons d'arbre 66 sont logés chacun dans un alésage d'une pièce solidaire de la chaussure et qui leur permet de tourner librement d'une valeur angulaire limitée, ce qui autorise un léger mouvement de roulis du pied et qui rend la course plus agréable à l'individu. Cependant, si celui-ci est équipé d'accessoires supplémentaires tels que patins à glace ou patins à roulettes à deux roues, il peut avoir intérêt à bloquer totalement ce mouvement de roulis avec une cale placée sous la pièce 64 par exemple. Comme dans tous les cas, l'amplitude de mouvement de roulis autorisé par l'appareil est faible, cela permet en terrain varié de préserver l'individu d'une fracture de la cheville grâce à la rigidité en torsion du bras 5 et au dispositif téléscopique.

La fixation des alésages recevant les tronçons d'arbre 66 sur la partie avant de la chaussure peut s'effectuer de nombreuses façons.

Les figures 35 et 36 représentent l'une d'elles qui est une pièce creuse métallique de préférence ou en plastique dur, entourant l'extrêmité d'une chassure ordinaire et fixée sous sa semelle. L'invention peut également comporter d'autres dispositions telles par exemple que la fixation de l'axe 63 au niveau de la semelle, surtout dans le cas où l'intéressé se sert généralement simultanément de patins et qu'il n'a pas besoin de mouvement de roulis. Enfin, l'axe 63 peut se fixer directement dans l'accessoire, patin ou ski, éventuellement utilisé. Par ailleurs, l'implantation de l'axe 63 par rapport à la longueur du pied est variable selon les usages. Elle tient compte aussi de la raideur en flexion de la chaussure. Le seul point commun est que cette implantation se trouve dans la partie avant du pied ou éventuellement devant celui-ci, afin de concerner la plus grande déformation géométrique possible pendant l'extension de la jambe et du pied qui causent la propulsion du corps.

La forme du bras de jonction 5d représentée en vue de dessus par rapport au pied sur la figure 37 et de profil sur la figure 2 est conçue en fonction des éléments suivants : compatibilité avec les encombrements et déplacements de la partie inférieure de la jambe et du pied, ce qui conduit à le disposer de préférence à l'extérieur des jambes et non pas entre les jambes et adaptation des sections à la valeur du moment fléchissant local. Par ailleurs, le bras 5 a de profil une forme de préférence courbe à concavité vers le sol afin de le rendre moins vulnérable aux obstacles. Le matériau constituant le bras de jonction 5 peut être un acier à hautes performances mécaniques ou un alliage léger ou un matériau composite à matrice organique, à condition que dans ces deux derniers cas, la jonction avec l'articulation 4 et notamment avec le rochet de blocage se fasse par une pièce rapportée en acier à hautes performances, l'axe 63 de l'articulation 6 devant également rester en acier.

Cette définition du bras de jonction 5 n'est pas limitative de l'invention et is se peut que les formes de celui-ci doivent être adaptées, soit à l'usage préférentiel d'un accessoire tel que patin, ski, etc ... soit à un type d'infirmité particulier.

Les figures 38 à 45 décrivent des modes de réalisation de l'articulation blocable et déblocable 4. L'articulation décrite est dans tous les cas une articulation droite de l'individu qui est symétrique de l'articulation gauche.

L'implantation générale, sur le dispositif téléscopique 3, de l'articulation 4 est décrite sur les figures 2, 3, 4, 5, 37, 51, 56, 58, 59, 60, 61. Cependant, cette implantation n'est pas limitative de l'invention car il est évident qu'on peut d'une part placer l'articulation 4 légèrement au-dessus de l'extrêmité inférieure du système téléscopique et d'autre part, la placer plus en avant ou plus en arrière, par rapport au sens de la marche de l'individu, de la position dans lequelle elle est représentée.

Dans ces cas et si les dispositifs téléscopiques 3 restent à la même place derrière les jambes, le bras de jonction 5 serait simplement plus court ou plus long et la partie de l'articulation 4 solidaire du dispositif téléscopique serait reliée à celui-ci par un bras rigide. On peut aussi, avec une forme de bras de jonction appropriée, utiliser une articulation en U traversé par un axe, dans lequel l'extrêmité du bras 5 forme le U et où le dispositif téléscopique se trouve au centre du U. Toutes sortes de variantes constructives sont donc possibles dans le cadre de l'invention globale.

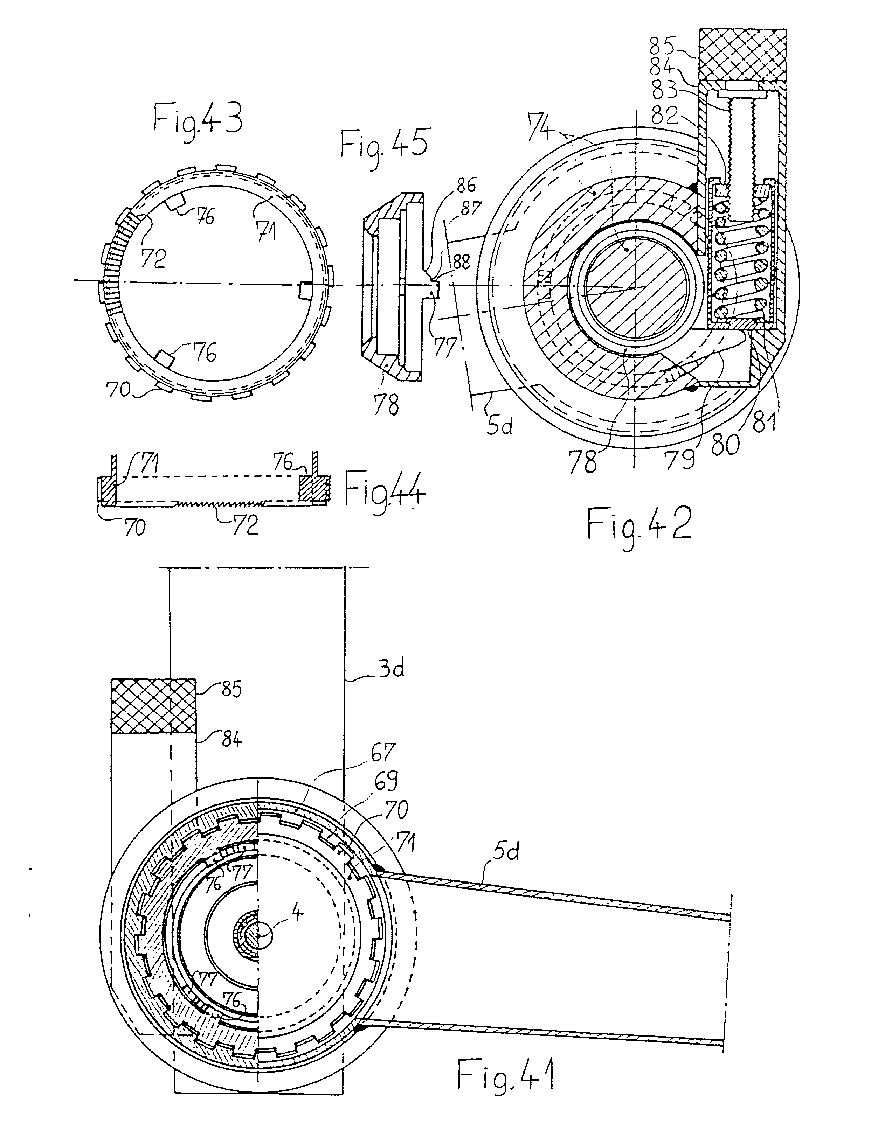

Sur les figures 38 à 40 sont décrites les articulations blocables 4. Celles-ci ont des parties communes, décrites aussi sur les figures 41 à 45 et ne diffèrent que par le mode d'actionnement du déblocage. Certains rôles de l'articulation 4 ont déjà été énoncés plus haut mais ils sont répétés et complétés ci-dessous :

L'articulation 4 permet, lors de la descente du pied qu'accompagne la descente du bras de jonction 5 et l'ouverture de l'angle que fait le bras 5 avec le dispositif téléscopique 3, d'interdire à tout moment le libre mouvement angulaire inverse grâce à un rochet poussé par un ressort.

Lorsque le pied est posé en avant du corps sur le sol et jusqu'à ce que tout effort ait cessé, c'est-à-dire à peu près jusqu'au décollement du sol du pied situé à l'arrière, l'articulation 4 reste bloquée et tous les efforts subis ou exercés par le système téléscopique transitent par elle. A la cessation des efforts, l'articulation 4 doit permettre la remontée instantanée du pied donc la diminution de l'angle que fait le bras de jonction 5 avec le dispositif télscopique 3. Pour cela, il faut donc que le système à rochet soit mis provisoirement hors service.

Par ailleurs, l'articulation 4 est munie d'une butée réglable qui permet de régler l'angle d'ouverture maximal entre le bras de jonction 5 et le système téléscopique 3 afin que, lorsque cet angle est atteint, l'effort que fait le pied en s'abaissant vers l'avant se transmette aux dispositifs téléscopiques 3 et allonge ensuite ceux-ci tout en comprimant leur ressort de rappel. Le réglage de la butée permet entre autres choses d'adapter dans une certaine mesure l'appareil à la taille d'un individu.

Cependant, aussi bien pour permettre un meilleur confort à l'individu, que pour éviter des chocs mécaniques brutaux, la butée est munie d'un ressort précontraint.

Pour comprimer davantage ce ressort avec la butée, la force descendante à appliquer par le pied par l'intermédiaire de l'articulation 6 est supérieure à celle qui a été nécessaire pour mettre le téléscope en extension maximale et comprimer à fond son ressort de rappel. La compression du ressort de butée représente donc un certain dépassement de l'angle maximal entre le bras 5 et le téléscope 3, prévu par le réglage de la butée, ce dépassement ne pouvant avoir lieu que si le dispositif téléscopique 3 est déjà en position d'extension maximale.

Un autre rôle de l'articulation 4 est de faire en sorte que le système à rochet ne puisse pas fonctionner pendant que le ressort de butée est comprimé et que par conséquent, l'ouverture de l'angle du bras 5 et du téléscope 3 dépasse une valeur prévue par le réglage de butée choisi. Il résulte de ce dernier rôle des avantages concernant l'adaptabilité de l'appareil selon l'invention à diverses sortes de reliefs, diverses vitesses, divers types de course avec ou sans bondissement et divers types d'accessoires supplémentaires tèls que patins, etc ... En effet, ce dernier perfectionnement permet dans certains cas de faire cesser l'assistance mécanique à la propulsion de la jambe en moyenne un peu avant la fin de la foulée arrière, si on le désire, et de terminer la foulée sans assistance mécanique. De cette façon, la fin de la foulée est ressentie de manière plus douce et plus naturelle par l'individu.

Par ailleurs, et notamment si l'axe 11 de l'articulation sous la selle est situé assez en arrière de celle-ci, l'allongement du dispositif téléscopique pendant la foulée avant d'un bond en terrain plat est généralement un peu plus grand que pendant la foulée arrière. Mais, grâce à la mise hors service du rochet pendant l'écrasement du ressort de butée, cet aspect n'est pas un inconvénient si la différence des valeurs d'allongement est absorbée par la course angulaire du bras de jonction 5 au-delà de la mise en butée.

Les moyens permettant à l'articulation blocable 4 de remplir les rôles qui lui sont assignés sont les suivants, mais ceux-ci ne sont pas limitatifs de l'invention. D'autres systèmes de rochet peuvent être mis en oeuvre ainsi que d'autres systèmes de mise hors service du rochet. Par ailleurs, le système de butée réglable et amortissante pouvant aussi mettre le rochet hors service est un perfectionnement très utile mais non absolument obligatoire. Le seul aspect obligatoire est de disposer d'un moyen de blocage et de déblocage de cette articulation.

Les parties communes aux différents types d'articulation 4 décrits seront étudiées sur les figures 39, 41, 42, 43, 44 et 45.

Sur la figure 39 on voit que le bras 5d est raccordé, par exemple par soudure à une embase cylindrique 67 munie d'une plaque d'obturation 68. On voit aussi en coupe cette embase 67 sur la figure 41. L'embase 67 comporte une collerette sur laquelle vient prendre appui un écrou 67a vissé sur l'autre partie de l'articulation, mais non bloqué, et freiné par la vis 67b. Comme on le voit sur la figure 37, il est avantageux que le plan de l'articulation 4 passe par l'articulation 6 car ainsi tous les efforts mécaniques resteront dans ce plan. L'embase 67 est munie d'une denture axiale 69 qui coopère avec une denture axiale 70 du rochet cylindrique 71 visible sur les figures 39, 41, 43, 44. Le rochet 71 peut donc coulisser axialement dans l'embase cylindrique 67 et lui transmettre un couple très élevé en l'absence de mouvements axiaux. Le rochet cylindrique 71 est également porteur, sur une face d'extrêmité d'une denture radiale à profil disymétrique 72 qui coopère avec une denture identique 73 taillée sur l'embase 74 de l'articulation 4 qui est solidaire, par soudure par exemple, de la partie inférieure 3i du dispositif téléscopique. La disymétrie des dentures 72 et 73 maintenues en contact par le ressort 75 qui exerce une poussée axiale sur le rochet et son prolongement autorise la rotation de l'articulation 4 dans un sens et non dans l'autre comme il est expliqué plus haut : le bras 5d peut donc s'abaisser, l'individu étant debout, et ne peut pas remonter.

Il est à noter que les dentures axiale 69 pourraient être solidaires de l'embase 74 et que les dentures radiales 73 pourraient être au contraire solidaire de l'embase 67. Cette disposition est du domaine de l'invention.

Un autre rôle du rochet cylindrique 71 est de transmettre les efforts de mise en butée angulaire de l'articulation 4 grâce aux trois aspérités 76. Celles-ci coopèrent avec trois aspérités 77 d'une pièce 78 visible sur les figures 39 et 45 à section générale circulaire qui peut tourner librement dans l'embase 74 à laquelle elle est liée axialement par l'écrou 78a. Le rochet circulaire 71 tend donc à entraîner en rotation la pièce 78 dès que les aspérités 76 rencontrent les aspérités 77.

La pièce 78 est munie d'un ergot 79 visible sur les figures 39 et 42, dont le rôle est d'appuyer sur la butée 80 qui est une cage cylindrique maintenant le ressort de butée 81 en position de précontrainte. La base de ce ressort appuie sur un écrou 82 coopérant avec une tige filetée 83 à laquelle il communique la poussée de l'ergot 79, et cette tige 83 la transmet à la cage cylindrique 84 soudée sur l'embase 74. En manoeuvrant la tête moletée 85, on peut déplacer le point d'appui initial entre l'ergot 79 et la butée 80 vers le haut, ce qui influe sur la position angulaire de la mise en butée. La figure 42 représente la butée 80 en position maximale de réglage vers le bas.

La compression du ressort 81 qui cause le déplacement axial de l'écrou 82 par rapport à la butée 80 permet donc à la pièce 78 portant l'ergot 79 de continuer provisoirement sa rotation donc d'ouvrir davantage l'ange formé par le dispositif téléscopique 3 et le bras de jonction 5.

On a vu plus haut que la pièce 78 tourne librement dans l'embase 74. Par conséquent, lorsque les aspérités 76 du rochet cylindrique 71 vont venir en contact avec la base du plan incliné 86 des aspérités 77, la seule force de pression axiale exercée par le ressort 75 sur le rochet 71 suffira à l'entraînement en rotation de la pièce 78 par les trois aspérités 76 sans que celles-ci montent sur les plans inclinés 86. Cette rotation de la pièce 78 amènera son ergot 79 en contact avec la butée 80. A ce moment, la poussée axiale due au ressort 75 ne sera plus suffisante pour permettre l'entraînement en rotation de la pièce 78 et les trois aspérités 76 monteront sur les trois plans inclinés 86 jusqu'aux surfaces 87 et viendront alors en butée sur les plans perpendiculaires 88. La transmission d'un couple important par le rochet sur la butée sera alors possible à nouveau mais l'ascension des trois plans inclinés 86 par les trois aspérités 76 aura causé un déplacement axial au rochet cylindrique 71 de sorte que sa denture dissymètrique 72 sera désolidarisée de la denture dissymétrique 73 de l'embase 74. Ainsi le mouvement angulaire de l'articulation 4 sera posible dans les deux sens tant que l'argot 79 exercera une force importante sur la butée 80.

Le déblocage de l'articulation par déplacement axial du rochet cylindrique 71 peut aussi s'effectuer sans rapport avec la butée angulaire à partir d'un effort hydraulique ou électro-magnétique ou électro-mécanique avec des dispositifs qui sont décrits sur les figures 38 à 40. Ces dispositifs nécessitent une arrivée de fluide ou de courant électrique délivrés par un système de pilotage qui sera décrit plus loin. il est également possible de réaliser un système de déblocage entièrement mécanique et non piloté, qui, fondé sur la mesure de la flexion du bras de jonction 5 sous l'effet du couple débloque l'articulation 4 dès que l'effort de propulsion par les dispositifs téléscopiques 3 cesse, donc en fin de foulée arrière de l'individu.

Ce dispositif mécanique, qui est plus complexe que les trois autres, n'est pas décrit pour alléger le texte mais cette éventualité supplémentaire souligne que les dispositifs décrits ne sont pas limitatifs de l'invention.

La figure 38 indique un premier principe de génération de déplacement axial du rochet cylindrique 71. Ce dernier contient une solénoïde 89 qui, lorsqu'il est traversé par un courant continu, se déplace vers la droite autour du noyau ferreux 90 solidaire de la plaque d'obturation 68 de l'embase 67. Lorsque le courant est coupé, l'ensemble du solenoïde 89 et du rochet 71 sont ramenés vers la gauche par le ressort 75 et les dentures 72 et 73 sont à nouveau au contact. Le courant électrique est amené par une fil 91 qui provient par un trou 92 de la plaque d'extrêmité de la partie inférieure ou femelle 3i du dispositif téléscopique.

La figure 39 indique un second principe de génération de déplacement axial du rochet cylindrique 71. Un moteur électrique 93 à courant continu est fixé par sa cage extérieure sur l'embase 74. Le rotor 94 de celui-ci est solidaire d'un écrou 95 qui peut tourner dans des paliers lisses de la cage extérieure du moteur 93 mais qui ne peut se déplacer axialement par rapport à elle. Cet écrou tourne autour d'une tige filetée 96 qui est immobilisée en rotation par une goupille 97 prenant appui dans l'embase 74. La rotation de l'écrou 95 entraîne donc le déplacement axial de la tige filetée 96, laquelle contient un arbre 98 solidaire du rochet cylindrique 71 et constamment repoussé vers la gauche par un ressort 75. Lorsque le moteur est traversé par un courant continu d'un certain sens, le rochet 71 est donc poussé à fond vers la droite et les dentures 72 et 73 sont désaccouplées. Lorsque le moteur 93 est traversé par un courant de sens contraire au procédent, la tige filetée 96 est repoussée à gauche au maximum et le rochet 71 est repoussé vers la gauche par la seule force de son ressort 75. L'arrivée de courant se fait de la même manière que sur la figure 38.

La figure 40 indique un troisième principe de génération de déplacement axial du rochet cylindrique 71. Une pièce solidaire du rochet 71 constitue un piston 98a qui peut se déplacer sans un cylindre 99 solidaire de l'embase 74. Le déplacement vers la gauche s'effectue avec le ressort de rappel 75 du rochet. Le déplacement vers la droite s'effectue sous l'action d'une pousée hydraulique, soit directement dans le volume limité par le cylindre 99 et la tête du piston 98, soit par l'intermédiaire d'une vessie souple 100 pour avoir une meilleure étanchéité. L'arrivée de fluide a lieu par des chemins analogues à ceux empruntés par les fils électriques et qui seront décrits plus loin, en même temps que les moyens destinés à générer les courants ou les pressions fluides aux moments voulus.

L'effort nécessaire pour effecteur le déplacement axial du rochet cylindrique 71 vers la droite est très faible car ce déplacement n'a lieu qu'en l'absence de couple mécanique exercé sur l'articulation 4

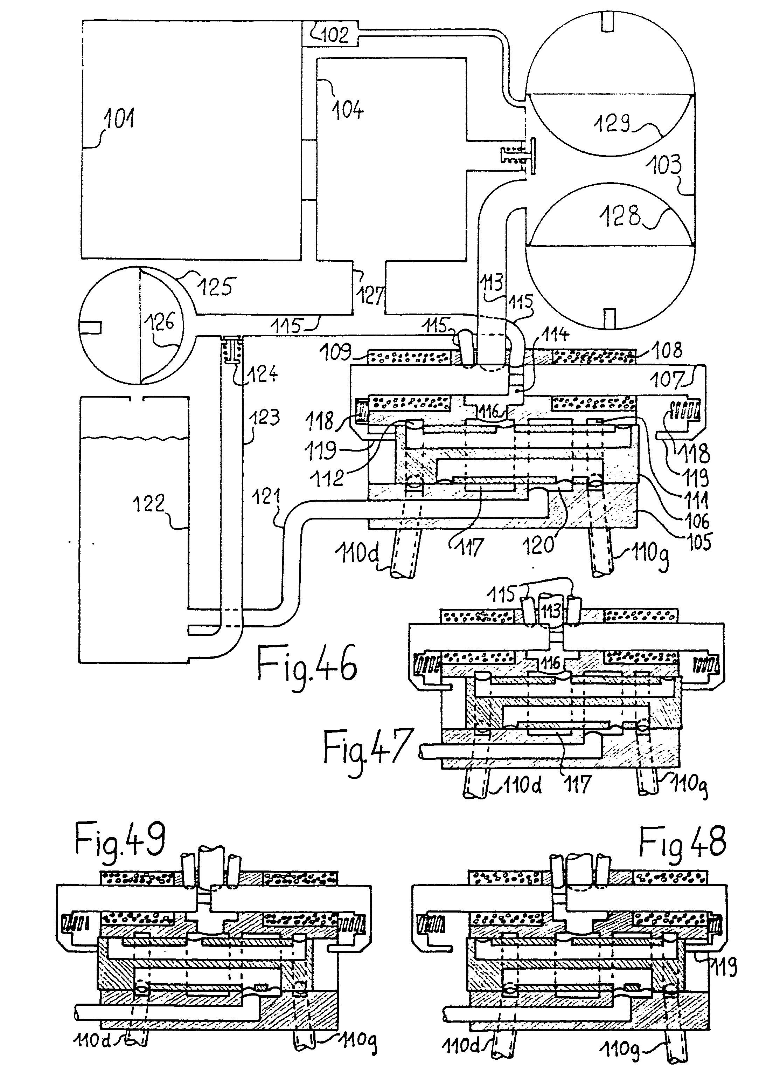

Les figures 46 à 50 décrivent des principes de génération et de distribution de fluides sous pression destinés à produire une force mécanique d'extension dans ceux des dispositifs téléscopiques 3 qui sont équipés d'une chambre étanche axialement extensible.

Sur ces schémas les échelles respectives des composants ne sont pas représentées afin de mieux décrire les aspects qui sont les plus spécifiques de l'application à l'invention en objet. Ces aspects n'en sont pas cependant limitatifs.

Pour les autres aspects, les deux systèmes globaux décrits font appel à un compresseur volumétrique et à un moteur thermique commandé par un régulateur qui asservit la puissance demandée à la pression moyenne que le pilote désire avoir dans un réservoir tampon. Un petit générateur électrique est associé au moteur thermique. Tous ces éléments sont bien connus de l'homme de l'art, ne sont pas limitatifs de l'invention et ne seront pas décrits ici, bien qu'il existe en ce qui les concerne des dispositions particulièrement favorables à l'usage de l'invention en objet. Il est d'une part, particulièrement souhaitable pour des raisons de légèreté que la plus grande partie de la structure résistante du moteur et du compresseur soit confondue et que les embiellages soient évités, grâce à des pistons libres, ou réduits au minimum. Les systèmes de refroidissement et d'isolation thermique seront également communs.. Enfin les moteurs à combustion externe à cycle de Stirling par exemple, seront quoique légèrement plus lourds, très préférables aux moteurs à combustion interne qui sont beaucoup plus bruyants.

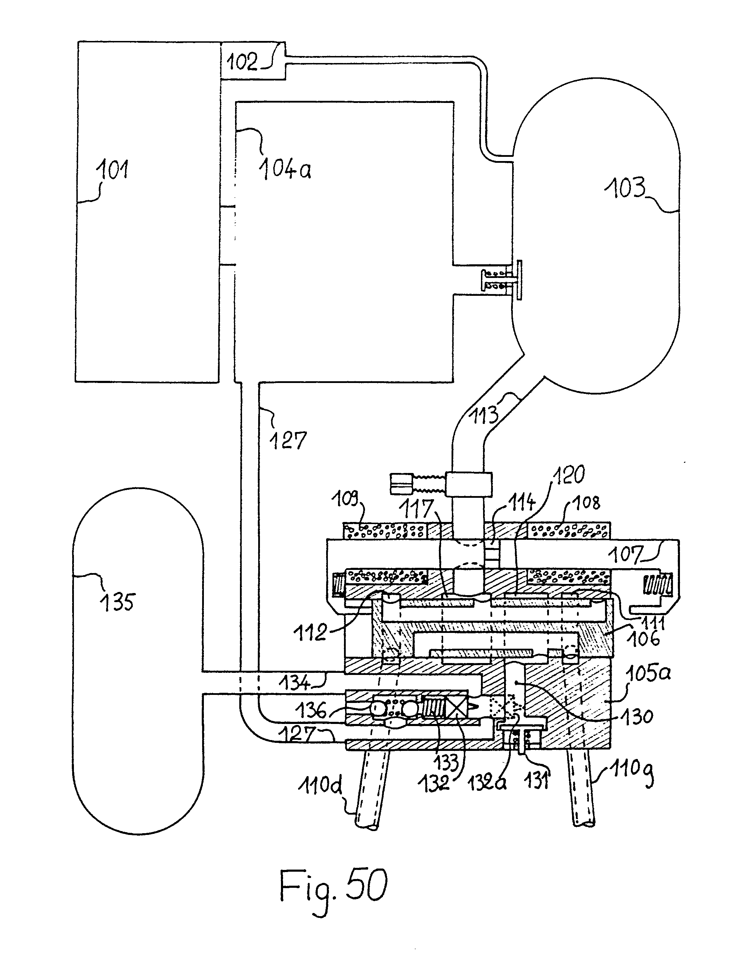

Le système décrit sur les figures 46 à 49 utilise un liquide comme fluide de transfert et le système de la figure 50 utilise un gaz qui est, à priori, de l'air. L'invention n'est limitée par l'emploi d'aucun liquide ou gaz particulier, y compris éventuellement un gaz de combustion.

Sur la figure 46 on distingue le moteur 101, le régulateur 102 asservi à la pression du réservoir tampon 103 et le compresseur volumétrique 104.

La puissance motrice est très variable selon les utilisations préférentiellement envisagées pour l'appareil mais elle se situe en principe dans la gamme de 1 à 10 KW, la majorité des applications nécessitant 2 à 5 KW. Pour ces puissances, et à titre purement indicatif, un groupe moto-compresseur performant, avec les réservoirs et le distributeur, pèsera de 4 à 8 kg environ sans le carburant, ce qui est l'ordre de grandeur de masse des tronçonneuses forestières manuelles. Dans le cas de l'invention, cette masse étant fixée au corps est très facile à transporter.

L'ordre de grandeur des pressions maximales de sortie du compresseur est en général dans la gamme 5 à 50 bars selon la section des vérins utilisés et le type d'usage recherché, de sorte qu'un compresseur de type volumétrique est normalement nécessaire. Ces pressions ne posent aucun problème pour l'emploi de canalisations souples, dont la nature bien connue de l'homme de l'art n'a pas à être décrite et les précisions d'usinage nécessaires pour les tiroirs de commande sont de valeur très courante en matériel hydraulique.

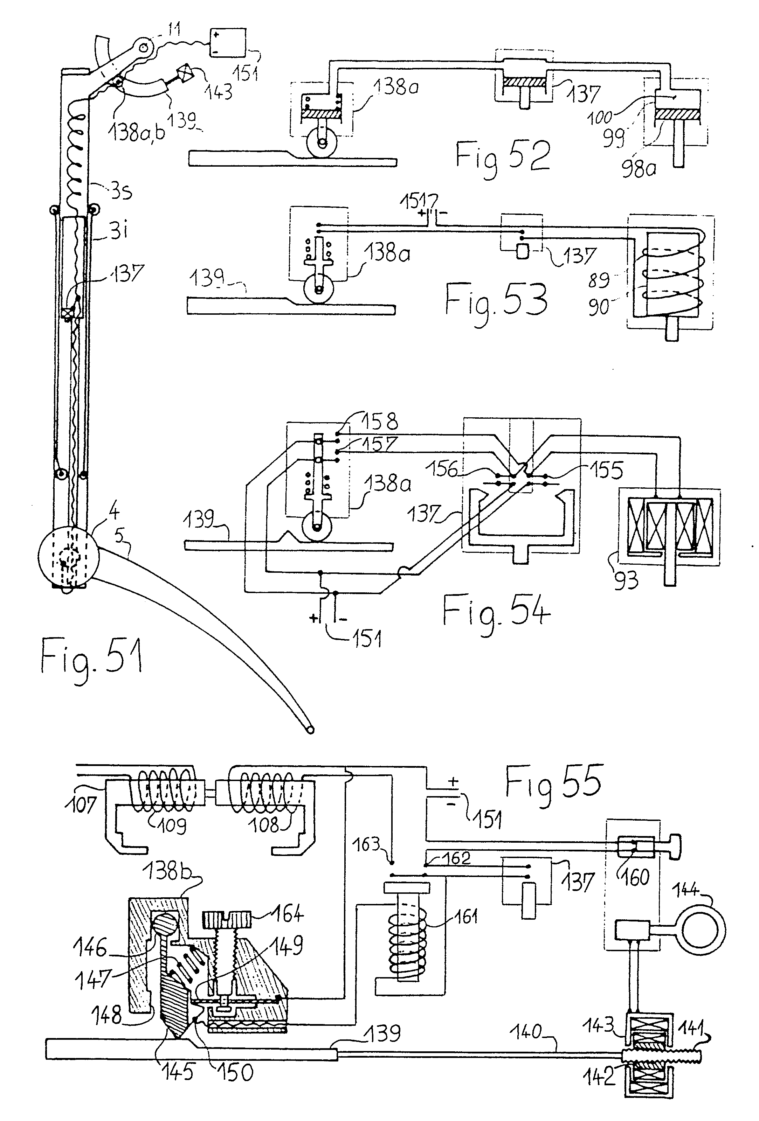

On distingue aussi un distributeur 105 comportant deux tiroirs 106 et 107. Ce système de distribution commun aux dispositifs téléscopiques 3 gauche et droit, a été choisi dans cette description parce qu'il permet de représenter de façon compacte l'ensemble des mouvements de fluide dans l'appareil pendant son fonctionnement. Mais dans la pratique, il y a plutôt intérêt à séparer les vannes ou tiroirs d'alimentation et vidange concernant chacun des dispositifs téléscopiques 3 car cela permet d'obtenir plus facilement les fortes sections de passage nécessaires pour avoir de faibles pertes de charge. Le système de pilotage demeure toujours voisin de celui de la figure 55.

Ce distributeur 105, schématiquement représenté, est actionné tour à tour dans un sens et dans l'autre par les solénoïdes 108 et 109 respectivement.

Les canalisations 110 g et 110 d relient le distributeur à la chambre étanche déformable contenue dans le système téléscopique gauche et droit respectivement. Elles aboutissent aux gorges 111 et 112 pouvant communiquer avec le tiroir 106.

La canalisation 113 relie le réservoir tampon 103 à la paroi du cylindre dans lequel coulisse le tiroir 107. Ce dernier possède une gorge 114 qui peut être mise, lorsqu'il occupe ses positions axiales extrêmes en relation avec les canalisations 115 reliées ensemble et lorsqu'il occupe ses deux positions axiales intermédiaires, en relation avec la canalisation 113. Dans tous les cas, la gorge 114 est reliée par le passage 116 à la gorge 117 pouvant communiquer avec le tiroir 106 Le tiroir 107 est muni à chacune de ses extrêmités d'un ressort 118 dont la longueur en l'absence de contact est connue avec précision et d'une aspérité 119 qui sert à pousser dans un sens ou dans l'autre le tiroir 106.

Une quatrième gorge 120 entoure le tiroir 106 et peut communiquer avec lui. Elle est reliée par la canalisation 121 à un réservoir 122 qui est à la pression atmosphérique. De ce réservoir part une canalisation 123 qui aboutit dans la canalisation 115 par une soupape anti-retour 124. La canalisation 115 est aussi reliée à l'entrée d'un réservoir d'amortissement et récupération partielle d'énergie 125 qui possède une membrane déformable 126 derrière laquelle se trouve de l'air comprimé.

Enfin, la canalisation 115 est reliée par la canalisation 127 à l'entrée du compresseur 104.

Le fonctionnement général, et du distributeur en particulier, sera mieux compris en considérant les quatre vues 46 à 49, d'abord pour un mode d'utilisation de l'appareil s'apparentant à celui décrit sur les figures 12 à 17, ensuite pour un mode d'utilisation s'apparentant à celui décrit sur les figures 18 à 20.

La figure 12 correspond à la position des tiroirs 106 et 107 représentée sur la figure 46 : la canalisation 110 g reçoit le liquide provenant du téléscope gauche 3g et le tiroir 106 l'envoit par la canalisation 121 dans le réservoir à pression atmosphérique 122 n'opposant donc aucune résistance à la remontée du téléscope gauche qui va se poursuivre sur les figures 13, 14 et jusqu'à la figure 15 sous l'action de son ressort de rappel 39 ou 61.

La canalisation 110d qui a permis d'envoyer dans le téléscope droit 3d pendant son allongement préalable à la figure 12 le fluide provenant du réservoir 122 via la canalisation 123, la soupape anti-retour 124, la canalisation 115, la gorge 114, le passage 116, la gorge 117, le tiroir 106 et la gorge 112, commence dès la figure 12 et jusqu'à la figure 15 à recevoir le fluide qui provient du téléscope droit qui se raccourcit et à l'envoyer dans la canalisation 115 où une partie sera aspirée par le compresseur par la canalisation 127 et où une autre partie ira dans le réservoir d'amortissement-récupération 125 où elle comprimera l'air comprimé en déformant la membrane 126. Le ressort à air comprimé a donc pour rôle d'amortir l'atterrissage et de stocker la quantité de fluide sous pression brutalement refoulée par le téléscope 3d que ne pourrait pas pomper assez vite le compresseur, mais qu'il pompera juste après.

La figure 15 correspond au moment du passage du tiroir 107 de la position qu'il occupait sur la figure 46 à celle qu'il occupe sur la figure 47. Il y a eu simplement coupure du courant de l'électroaimant 108 qui maintenait comprimé le ressort 118 qui est à gauche sur la figure. Celui-ci en se détendant a causé le déplacement du tiroir 107 sans déplacer le tiroir 106. Ainsi le passage 116 et la gorge 117 ne sont plus en relation avec la canalisation 115 mais avec la canalisation 113 qui permet d'envoyer dans le téléscope 3d le liquide sous pression qui procure à l'individu la force motrice d'assistance à la propulsion par sa jambe droite.

Le réservoir tampon 103 peut contenir, au besoin et très schématiquement, deux membranes déformables 128 et 129 derrière lesquelles se trouvent respectivement de l'air comprimé et un gaz ou mélange de gaz de la famille des fréons par exemple, qui est ou qui sont dans des conditions de température et de pression très proches de leur équilibre liquide-gaz. Ainsi, le rôle de l'air est de servir de ressort de stockage d'énergie selon la loi $PV = RT$, ce qui conduit, le réservoir tampon 103 étant petit car nécessairement léger, à une évolution décroissante de la pression qui s'exerce sur les pistons des téléscopes au fur et à mesure de leur allongement. Le rôle des mélanges de fréons ou autres gaz proches des conditions de température et de pression de leur équilibre liquide-gaz est d'atténuer les écarts de pression constatés en fonction de l'allongementdes téléscopes. Ainsi, selon les usages préférentiels qui sont prévus pour un certain type d'appareil selon l'invention, on peut mettre en jeu la loi de variation de pression la plus souhaitable pour le confort et l'efficacité.

La figure 16, qui est la fin de la foulée propulsive par la jambe droite correspond au passage à la position des tiroirs représentée sur la figure 48. Le solénoïde 109 déplace à fond le tiroir 107, ce qui d'une part déplace le tiroir 106 par action de la pointe 119, d'autre part, comprime à fond le ressort 118 qui est à droite de la figure et enfin met la gorge 114 en relation avec la canalisation 115. Cela permet comme on le voit la vidange du fluide contenu dans le téléscope droit dont la position d'extension extrême est atteinte et son envoi par la canalisation 110d, la gorge 120, le tiroir 106 et la canalisation 121 dans le réservoir 122. Parallèlement le liquide du réservoir 122 via la canalisation 123, la soupape 124, la canalisation 115, la gorge 114, le passage 116, la gorge 117, le tiroir 106 et la canalisation 110g va dans le téléscope gauche dont le volume s'accroît puisqu'il s'allonge ainsi que sur la figure 17.

La figure 49 est la dernière des positions possibles des tiroirs suite à la coupure du courant dans le solénoïde 109. Elle correspond à la figure 47 en inversant les positions des téléscopes droit et gauche.

La figure 18 qui correspond au début d'application du poids du corps sur la jambe droite est la moment où le distributeur passe, par coupure du courant électrique, de la position de la figure 46 à celle de la figure 47. Le seul changement est la détente du ressort 118 situé à gauche du tiroir 107 sur la figure, ce qui met la gorge 114 de ce tiroir en relation avec la canalisation 113 et obture la canalisation 120. Ainsi, le dispositif téléscopique de la jambe droite est alimenté par le liquide sous pression du réservoir tampon. Parallèlement, la position du tiroir 106 n'a pas bougé par rapport à la position de la figure 46 donc la vidange du liquide contenu dans le dispositif téléscopique 3g de la jambe gauche peut se poursuivre en direction du réservoir 122.

La figure 19 correspond à la position du distributeur que l'on voit sur la figure 47.

A la fin du mouvement représenté sur la figure 20, c'est-à-dire de l'extension maximale de la jambe droite, la position du distributeur devient celle que l'on voit sur la figure 48. A ce moment, le pied gauche est déjà en contact avec le sol, mais il ne supporte pas encore le poids du corps. Un instant plus tard, le poids du corps se porte sur le pied gauche dans une position identique à celle décrite sur la figure 18 pour le pied droit, et à ce moment, le distributeur prend la position décrite sur la figure 49, ce qui admet la pression motrice dans le dispositif téléscopique 3g de la jambe gauche.

Ainsi, on voit que le système décrit sur les figures 46 à 49 permet d'assurer la distribution de puissance : d'une part, suivant la loi de variation de pression désirée grâce au réservoir tampon 103 et à ses annexes à air ou gaz parfait 128 ou à gaz proche de l'équilibre gaz-liquide 129. D'autre part, en amortissant la retombée sur le pied avant, en stockant l'énergie et en étalant la durée de pompage à débit maximal du compresseur 104 grâce au réservoir 122 contenant un réservoir d'air ou de gaz parfait 126. Enfin, en s'appliquant à tous les cycles possibles de déplacement qui vont de la course avec bonds à la montée de pentes pendant laquelle le fléchissement maximal de la jambe avant est atteint dès le moment où elle se pose sur le sol. Le circuit hydraulique peut de plus être adapté à de multiples applications spécifiques. On pourra prévoir par exemple un équipement spécial permettant à certains infirmes de descendre les pentes fortes où les escaliers sans efforts musculaires de retenue dans les jambes. Il suffit de prévoir sur les canalisations 110 par exemple, une soupape permettant le remplissage rapide des téléscopes mais une vidange par une section très faible réglable à volonté.

Sur la figure 50 est représenté un principe de génération, distribution et récupération d'air comprimé. Si le gaz employé est l'air, les petites fuites sont sans importance, ce qui permet l'emploi d'un matériel moins coûteux.

En revanche, l'air comprimé ou n'importe quel gaz, laissent dans le volume étanche du téléscope en position d'extension maximale et avant la vidange permettant de remonter la jambe concernée, une importante quantité d'énergie de détente. Ce problème n'existe pas si le fluide est incompressible. Il est donc très utile de récupérer la majeure partie de cette énergie si on veut conserver un bon rendement de l'appareil. La présence de l'articulation déblocable 4 qui permet de remonter le pied instantanément, même si le dispositif téléscopique reste momentanément allongé au maximum, suffit à récupérer l'énergie de détente de l'air si on le réaspire directement dans le compreseur. Cependant, il peut être intéressant, dans certains cas d'emploi, de diminuer le temps de vidange du dispositif téléscopique, ce qui conduit à utiliser un réservoir intermédiaire de détente.

Par ailleurs, il est pratiquement impossible d'amortir la chute sur le pied avant au moyen de l'air contenu dans le dispositif à la suite de son remplissage à la pression atmosphérique. L'amortissement de la chute avant s'obtiendra en effectuant une admission prématurée d'air comprimé par exemple dans la position de la figure 13, grâce au système de pilotage que l'on verra plus loin.

Le système décrit sur la figure 50 comprend, ainsi que celui de la figure 46, un moteur 101, un régulateur 102, un compresseur volumétrique 104a envoyant l'air comprimé dans un réservoir tampon 103 pouvant être muni d'un réservoir de gaz à l'équilibre gaz-liquide séparé par une membrane souple 129 non représentée.

Le distributeur 105a est muni de deux tiroirs 106 et 107 semblables à ceux du distributeur 105 et fonctionnant de la même manière. Ce distributeur 105 a été décrit par analogie avec le système de la figure 46 mais on peut obtenir en pratique de meilleures sections de passage en séparant la distribution droite de la distribution gauche avec une électro-vanne à 3 voies chaque dispositif téléscopique reliées à la canalisation 110g ou 110d et tour à tour à la canalisation 113 ou 130.

Le distributeur 106 est en relation avec les dispositifs téléscopiques droit et gauche par les canalisations 110g et 110d respectivement, aboutissant aux gorges 111 et 112 situées autour de lui.

La gorge 117 est également réservée à l'alimentation lorsqu'elle est en relation avec le réservoir tampon 103 par la canalisation 113 et la gorge 120 est réservée à la vidange-récupération.

Les canalisations 115 étant supprimées dans le distributeur 105a, la gorge 114 du tiroir 107 n'est en communication avec aucun orifice lorsque ce tiroir occupe ses deux positions extrêmes et est en communication avec la canalisation 113 lorsqu'il occupe ses deux positions intermédiaires.

La figure 50 qui correspond, pour la disposition des tiroirs en relation avec la position des jambes, à la figure 46 ou à la figure 48 en inversant le nom des jambes, représente plus précisément le début de la vidange récupération du dispositif téléscopique 3g de la jambe gauche. A ce moment où le tiroir 107, sous l'action de l'électro-aimant 108 vient de déplacer le tiroir 106, la vidange commence et de l'air comprimé à pression élevée arrive au distributeur 105a par la canalisation 110g. Cet air est admis par le tiroir 106 dans la gorge 120 et la canalisation d'évacuation 130. Sa pression élevée ferme instantanément la valve 131 en comprimant son ressort et repousse à fond le piston 132 en comprimant son ressort 133. La position initiale 132a de ce piston est représentée en pointillés. Le recul du piston 132 a dégagé l'entrée de la canalisation 134 qui conduit l'air comprimé vers le réservoir intermédiaire de détente 135.

Parallèlement, un débit d'air est admis dans la canalisation 127 vers l'admission du compresseur. Cette double sortie de l'air permet donc un début de vidange très rapide du dispositif téléscopique, de sorte que la pression d'air comprimé chute immédiatement. De ce fait, le piston 132 poussé par le ressort 133 ravance, obture le conduit 134 en emprisonnant l'air comprimé du réservoir intermédiaire de détente 135 mais permettant encore un pompage par le compresseur dans la canalisation 127 ce qui fait encore baisser la pression d'air comprimé provenant de la canalisation 110g. De ce fait, la soupape 131 s'ouvre et le piston 132 revient à sa position initiale 132a. Il fait alors communiquer l'air comprimé du réservoir 135 avec l'aspiration du compresseur qui le vide rapidement. Lorsque le réservoir 135 est vide, le complément d'aspiration du compresseur s'effectue par la valve 136.

Le système décrit permet donc une vidange très rapide du dispositif téléscopique concerné, en récupérant, suite aux pertes et aux détentes intermédiaires de l'ordre des deux tiers de l'énergie de détente de l'air. Cependant, comme il est dit plus haut, ce système ou un système ayant le même but, n'est généralement pas nécessaire sauf s'il est obligatoire pour les applications visées pour l'appareil de se vidanger très vite les dispositifs téléscopiques. A l'exception de ces cas, on relie simplement la canalisation de vidange 130 à la canalisation 127 d'admission dans le compresseur et le rendement de la récupération s'en trouve amélioré.

Dans le système décrit sur la figure 50, il n'est pas prévu de conduit pour le remplissage d'air à la pression atmosphérique lorsque la jambe droite descend.

Ce remplissage s'effectue en effet plus avantageusement par une valve non représentée située au sommet des dispositifs téléscopiques, sous la selle. L'air ainsi aspiré et comprimé ensuite lors de la chute sur le pied avant droit apporte par ailleurs une participation mineure à l'amortissement mais celui-ci reste essentiellement fondé sur une admission prématurée d'air comprimé au gré de l'intéressé.

On aborde maintenant la description de l'ensemble du système de pilotage de l'appareil et de ses différentes fonctions partielles. Les schémas de principes sont représentés sur les figures 51 à 55 mais les explications doivent se référer également à d'autres figures. Par ailleurs, il est entendu que les systèmes décrits sont doubles : l'un concerne la partie gauche de l'appareil avec des interactions éventuelles sur la partie droite et réciproquement. Les systèmes choisis sont parmi les plus simples possibles applicables à un même appareil devant fonctionner pour des usages très variés. Bien entendu, il est possible de spécialiser les systèmes de pilotage en vue d'usages préférentiels très spécialisés de l'appareil dont quelques uns seront décrits dans le paragraphe concernant les variantes de l'invention.

L'appareil peut comprendre :
-Soit un système de pilotage entièrement automatique pendant le fonctionnement, certaines options le concernant pouvant être éventuellement choisies avant le départ,
-Soit un pilotage automatique avec correction manuelle des réglages pendant la marche,
-Soit un pilotage au moins partiellement manuel pendant la marche, réservé à des usages à rythme lent.

Pour tous les cas, des réglages peuvent être effectués à l'arrêt pour favoriser l'emploi de l'appareil dans un certain type de parcours. Ce sont par exemple le réglage de la vis de butée 85 de l'articulation 4 ou le réglage de la vis de butée électrique 164 située sur la commande du tiroir 107 du distributeur, ou les pressions gazeuses situées derrière les membranes souples 128, 129 des réservoirs tampon 103 ou du réservoir de stockage-récupération 125.

Les éléments ou fonctions de l'appareil qui doivent être pilotés sont au minimum, le déblocage de l'articulation 4, l'autorisation de reblocage de l'articulation 4, le remplissage et la vidange des volumes étanches et expansibles des dispositifs téléscopiques 3 si l'appareil en comporte et la pression du réservoir tampon 103 asservissant le régime du moteur si l'appareil en comporte un.

Le système complet de pilotage comporte :
-des capteurs de données physiques
-un système de traitement de ces données
-des dispositifs d'exécution mécanique des ordres reçus par le système
-un système de commandes accessibles à l'individu et les annexes qui le relient au système de traitement des données ou qui agissent directement sur la nature des données physiques
-des fils ou tubes de jonction entre tous les éléments précédents.

Le premier des capteurs est celui qui détecte l'arrivée en butée d'extension maximale des systèmes téléscopiques.

On voit sur la figure 32 un tel capteur 137 qui établit un contact électrique dès que le piston 49 poussé par le ressort précontraint 48 arrive en bout de course. Dans ce cas, le capteur 137 est pressé contre la roulette 45. Cette représentation est - schématique et nullement limitative car de nombreuses autres dispositions sont possibles. Le capteur 137 pourrait aussi être fixé par exemple sur la partie supérieure 3s du dispositif téléscopique et détecter une discontinuité mécanique ou autre, solidaire de la partie inférieure 3i de ce dispositif, etc ... Notons que le capteur 137 ou assimilé n'est pas représenté sur les figures 29 et 33 uniquement pour simplifier le dessin.

Par ailleurs, d'une part le capteur 137 ou assimilé peut éventuellement détecter une position d'extension du dispositif téléscopique 3 qui ne correspond pas à sa position d'extension maximale mais qui se situe chronologiquement un peu avant. D'autre part, le capteur 137 peut être remplacé par un système de détection permanent de la position d'extension du dispositif téléscopique 3. Un tel système peut être facilement logé par exemple dans la cavité existant à l'intérieur des pistons 55 et 36 comme on le voit sur les figures 33 et 29. Il peut consister par exemple en un système téléscopique muni de signaux magnétiques axialement disposés, ou mesurant une résistance électrique proportionnelle à l'allongement. La prise en compte des indications fournies par ce système de détection de la position d'extension, au même titre que celles d'un capteur de butée 137, permet pour certains usages d'assouplir le fonctionnement de l'appareil. En effet, la longueur de la partie assistée mécaniquement de l'extension du pied est définie par le réglage de la butée 80 avec la vis 85, éventuellement motorisable, mais la détection de l'allongement téléscopique est un autre moyen de régler la longueur de la partie motrice de l'extension et d'allonger progressivement la foulée lorsque l'on démarre. Il faut simplement adapter dans ce cas les circuits de pilotages décrits sur les figures 52 à 55.

Le capteur de type 137 ou assimilé peut d'une part déclencher un signal électrique, comme sur les schémas des figures 53 à 55 ou un signal hydraulique comme sur la figure 52, qui dans les deux cas commandent le déblocage de l'articulation 4 en fin de foulée propulsive pour permettre la remontée du pied.

D'autre part, et seulement s'il déclenche un signal électrique, il peut servir à la fois pour commander le déblocage de l'articulation 4 et la vidange du fluide contenu dans le dispositif téléscopique 3 si la force motrice vient d'un fluide comprimé. Toutefois, il peut être souhaitable que ces deux fonctions soient chronologiquement légèrement décalées et nécessitent par conséquent deux capteurs 137. L'un d'eux détectera par exemple la mise en butée d'extension maxi et l'autre détectera une position d'extension voisine de celle-ci. Ce décalage chronologique éventuel des ordres donnés peut d'ailleurs s'obtenir avec un seul capteur complété par exemple par un retard électronique.

Le deuxième système de détection est composé d'un ou plusieurs capteurs 138 se déplaçant sur une ou plusieurs pistes 139 composées d'une ou plusieurs fractions de couronne circulaire centrées sur l'axe de l'articulation 11 de la selle et fixées par exemple sur la cloison médiane de celle-ci. Ce deuxième système est visible sur les figures 21 à 28 et 51 à 55. Les capteurs 138 peuvent aussi se déplacer radialement sur la piste 139.

La représentation des pièces 138 et 139 qui est faite par exemple sur la figure 22 est très simplifiée. En effet, d'une part, la piste est unique alors qu'elle peut comporter plusieurs pistes concentriques, d'autre part elle n'indique pas de variation d'épaisseur, enfin le système de réglage de position angulaire n'est pas représenté. La raison est que les figures 21 à 28 sont effectuées pour des cas d'application générale comprenant aussi les versions les plus rudimentaires.

Le rôle de ce deuxième système de détection est de permettre de synchroniser certains ordres de pilotage soit avec une position angulaire particulière des dispositifs téléscopiques 3 avec la selle ou entre eux telle que, par exemple, le croisement des jambes représenté sur la figure 15, soit avec le déplacement angulaire extrême des jambes, ce dernier étant d'amplitude variable au gré de l'utilisateur.

Pour détecter une position angulaire particulière des dispositifs téléscopiques 3 par rapport à la selle ou entre eux on peut utiliser une différence d'épaisseur sur une certaine portion de la longueur circonférencielle concernée sur la piste 139 et visible sur les figures 52, 53, 55 où cette piste apparaît de profil et montre son épaisseur variable et sa longueur circonférencielle. Ainsi, la roulette ou le frotteur du capteur 138 ne portent sur la piste que dans la zône de forte épaisseur de celle-ci.

On peut aussi placer une aspérité géométrique sur la piste 139 comme sur la figure 54, de manière à ce qu'un signal soit enregistré par le capteur 138 lorsqu'il passe dessus.

Pour obtenir la variation de position angulaire du repère ou de la discontinuité d'épaisseur de la piste 139, on peut utiliser une gorge de guidage circulaire centrée sur l'axe de l'articulation 11, dans laquelle la piste 139 se déplace sous l'action, visible sur la figure 55, d'une tige 140 reliée à une vis 141 déplaçable axialement par un écrou 142 solidaire du rotor d'un moteur électrique pas à pas 143 commandé par un levier de pilotage 144.

Il est évident qu'on peut remplacer les différences de niveau ou aspérités précédemment décrites pour la piste 139 par une signalisation magnétique fixée sur celle-ci et coopérant avec un capteur 138 approprié, ces systèmes étant bien connus de l'homme de l'art.

Pour détecter le déplacement angulaire extrême d'une jambe, il faut détecter le point de changement de sens de circulation du capteur 138 sur la piste 139.

On peut y parvenir avec un capteur 138b du type décrit par la figure 55. Ce capteur 138 b possède un frotteur 145 qui comporte une extrêmité d'ancrage 146 qui, coopérant avec un logement approprié, solidaire du corps du capteur 138 b, lui permet de tourner légèrement dans le plan de la figure et de se déplacer verticalement vers le bas lorsqu'il quitte la partie la plus épaisse de la piste 139. Le frotteur 145 ne peut pas frotter toutefois sur la partie la moins épaisse de la piste 139. Le frotteur 145 est poussé par un ressort oblique 147 qui, prenant appui sur le corps du capteur 138b exerce sur lui une force vers le bas et vers la gauche de la figure 55. Ainsi, si le capteur 138b se déplace vers la gauche de la figure 55, une force verticale d'appui sur la piste 139 et donnée au frotteur 145 par le ressort oblique 147 dès que celui-ci monte sur la partie épaisse de la piste 139. Mais la force tangentielle de frottement qui en résulte durant le déplacement tend à éloigner le frotteur 145 de sa butée gauche 148, ce qui a pour effet de comprimer davantage le ressort 147. Dans cette position, le frotteur 145 vient en appui sur le conducteur électrique 149 ce qui établit le contact avec le fil souple 150 qui est soudé au frotteur 145 dans la seule zône conductrice de celui-ci.

Si l'ensemble du capteur 138b se déplace maintenant vers la droite de la figure 55, cela correspond à un changement de sens du déplacement angulaire de la jambe et du dispositif téléscopique qui y est relié. Dans ce cas la force tangentielle de frottement sur la piste 139 tend à faire venir le frotteur 145 en contact avec sa butée 148 et à couper le contact électrique. Les autres fonctions du capteur 138b de la figure 55 seront étudiées plus loin.

Il existe d'autres moyens pour détecter le déplacement angulaire maximal du capteur 138b sur la piste 139. Par exemple, cet ensemble peut être transformé en rhéostat électrique comprenant un frotteur isolé et une piste 139 isolée électriquement de la cloison médiane 19 de la selle 1, jouant alors le rôle de résistance variable. Il est alors facile de déterminer électriquement en permanence la valeur de la dérivée mathématique d'un courant parcourant le système. La dérivée nulle correspond alors au point de changement de sens. Ce système rhéostatique permet également de supprimer les discontinuités géométriques sur la piste 139 puisqu'il suffit de les remplacer par une mesure de la résistance électrique du circuit. Cela simplifie également le pilotage car on peut éviter de déplacer la piste 139 pour modifier la position angulaire du repère. Il suffit que le conducteur affiche une valeur de son choix pour la nouvelle résistance critique du système rhéostatique.

L'implantation générale des capteurs et détecteurs 137 et 138 est visible sur la figure 51.

On voit tout d'abord qu'il existe une ou plusieurs liaisons électriques par fil souple entre l'emplacement du capteur 138 et une batterie 151 ou une source de courant provenant du groupe motocompresseur éventuel.

Il existe ensuite une liaison électrique ou hydraulique entre l'emplacement du capteur 138 et celui du capteur 137. Il existe enfin une liaison électrique ou hydraulique entre le capteur 137 et le fond de la partie inférieure 3i du dispositif téléscopique 3. Après être sortie du fond du dispositif téléscopique inférieur 3i, cette liaison pénètre dans l'embase fixe 74 de l'articulation 4 par le trou 92 donnant accès au conduit 91 par où elle est reliée au dispositif électro-magnétique, électro-mécanique ou hydraulique qui commande le déplacement axial permettant la mise hors service du rochet cylindrique 71.

La liaison entre le capteur 138, le capteur 137 et l'articulation 4 peut s'effectuer dès deux façons suivantes, compte tenu du mouvement téléscopique entre les parties 3i et 3s.

D'une part, ainsi qu'on le voit sur les figures 29, 31, et 32, la liaison partant de l'emplacement du capteur 138 peut être un fil ou conduit tubulaire souple hélicoïdal 152 disposé à l'intérieur de la partie supérieure 3s du dispositif téléscopique, pénétrant dans la tête du piston solidaire de la partie inférieure 3i du dispositif téléscopique, reliant le capteur 137, et descendant ensuite jusqu'à la base de la partie inférieure 3i par un conduit foré dans la tige du piston 36r ou 49r.

D'autre part, ainsi qu'on le voit sur les figures 33 et 34, la liaison partant de l'emplacement du capteur 138 peut être un fil ou conduit tubulaire souple 153 descendant dans une rainure longitudinale, non visible sur les schémas, de la partie supérieure 3s du dispositif téléscopique jusqu'à sa jonction avec la pièce 53 où ce fil sort de sa rainure est relié à un capteur 137 et pénètre ensuite dans un prolongement inférieur cylindrique creux 154 de la pièce d'appui 58 du ressort de rappel 61, passant entre les roulettes 50 et 51. Ensuite le fil ou conduit souple 153 prend la forme d'une hélicoïde et descend à l'intérieur du tube fendu 57 jusqu'en bas de la partie inférieure 3i du dispositif téléscopique.

Ces deux méthodes ne sont pas limitatives mais le procédé de jonction choisi doit toujours tenir compte des mouvements de coulissement téléscopiques ce qui n'est pas très difficile comme on le voit.

Les types et principes de capteurs détecteurs décrits ne sont pas non plus limitatifs de l'invention. En particulier, il peut être envisagé de s'intéresser à des signaux physiques complémentaires ou de remplacement dans le cas où l'invention ou ses variantes sont appliquées à des cas d'emploi très spécifiques.

La première fonction à remplir par les éléments du système automatique de pilotage est le déblocage et l'autorisation de reblocage de l'articulation 4. Le déblocage de cette articulation a toujours lieu vers la fin de la foulée propulsive de la jambe concernée et par conséquent, à la fin de la course d'extension du dispositif téléscopique 3 ou presqu'à la fin de celle-ci. On a vu plus haut que le conducteur de l'appareil dispose d'une possibilité d'adaptation mutuelle de ces élements en fonction du terrain ou de la vitesse grâce à la vis 85 de réglage de butée de l'articulation 4 et à l'éventualité de pouvoir détecter un signal légèrement précurseur de la mise en butée d'extension du dispositif téléscopique 3. Le déblocage de l'articulation a lieu à la fin du mouvement représenté sur la figure 16 ou la figure 20.

L'autorisation de reblocage, c'est-à-dire l'aptitude pour le rochet cylindrique 71 à empêcher la remontée du bras 5 après que celui-ci soit descendu d'un angle quelconque inférieur ou égal toutefois à l'angle défini par la vis de butée 85, est donnée au moment où la remontée du pied qui va le premier se reposer sur le sol est maximale. Cela conduit pratiquement à donner cette autorisation à peu près au moment où le pied qui va se poser au sol dépasse en allant vers l'avant le pied qui est au sol, donc dans la position des figures 15 et 19 approximativement.

Pour effectuer ces deux fonctions de déblocage et autorisation de reblocage de l'articulation 4, on utilise les circuits hydrauliques ou électriques suivants :

- Si le moyen pour exercer une force d'extension dans le dispositif téléscopique 3 est un ressort précontraint et comprimé 48, les circuits sont décrits sur les figures 52 et 53.

Sur la figure 52, on voit que la roulette du capteur 138 a qui agit sur un piston hydraulique ou une vessie souple, peut se déplacer, lorsque le pied est en avant du corps par rapport au sens de la marche sur la partie la moins épaisse de la piste 139 et, lorsque le pied est en arrière du corps sur la partie la plus épaisse de la piste 139. Lorsque la jambe est dans la position représentée sur les figures 16 et 20, la quantité de liquide contenue dans le capteur 138a est donc minimale. Si à ce moment, le capteur de butée 137 vient en butée, son piston hydraulique ou sa vessie souple éjectent une quantité de liquide qui ne peut donc se rendre que dans la cavité 100 et pousser le piston 98a dans le cylindre 99. Cela cause le déblocage de l'articulation 4 comme on le voit sur la figure 40. La précontrainte du ressort 48 s'exerçant en permanence, l'articulation 4 reste débloquée pendant toute la remontée du pied concerné. Mais, lorsque ce pied dépasse celui qui est alors au sol dans la configuration des figures 15 et 19, en inversant sur ces figures la jambe droite et la gauche, la roulette du capteur 138a tombe sur la partie la moins épaisse de la piste 139. Cela libère un volume occupable par le liquide et permet, sous l'influence du ressort du rochet 75 la vidange du cylindre 99 et le recu du piston 98a. Ainsi le rochet 71 est à nouveau apte à fonctionner. Lors du blocage de l'articulation 4, après atterrissage du pied avant, une force de compression s'exerce sur le ressort 48, ce qui supprime l'effort de compression sur le capteur 137 et qui libère dans celui-ci un espace qu'occupera la fluide contenu dans le capteur 138a lorsque celui-ci remontera sur la partie épaisse de la piste 139 et ainsi de suite.

Sur la figure 53 est représenté un système électrique avec batterie dont le principe est absolument analogue à celui du système hydraulique. Lorsque le capteur de butée 137 établit un contact sur le circuit, le contact du capteur 138a est établi puisque la roulette est sur la partie épaisse de la piste 139, donc l'électro-aimant 89 prenant appui sur le noyau 90 débloque l'articulation 4 comme on le voit sur la figure 38. L'action de cet électro-aimant est supprimée par la rupture du contact 138a après le croisement des pieds ce qui rétablit le rôle initial du rochet 71.

-Si le moyen pour exercer une force d'extension dans le dispositif téléscopique 3 est une pression fluide, le circuit est décrit sur la figure 54 qui se réfère à la figure 39 en ce qui concerne le moyen mécanique de déblocage qui est un moteur électrique 93 agissant sur un écrou axial 95 et une vis axiale 96.

Dans ce cas, le mécanisme de la commande consiste à envoyer un courant continu d'un premier sens dans le moteur pour obtenir le déblocage de l'articulation 4 et un courant continu de sens inverse pour obtenir l'autorisation de reblocage de celle-ci.

La piste 139 possède seulement une aspérité correspondant à la position de la roulette du capteur 138a au moment où le pied arrière qui est levé dépasse en allant vers l'avant le pied qui est au sol.

Sur la figure 54, on voit que lorsque le capteur 137 détecte la position de butée suite à l'extension extrême du dispositif téléscopique 3, il établit un contact électrique double en 155 et 156 qui envoie un courant continu d'un premier sens dans le moteur 93 et débloque l'articulation 4.

Si la force cesse sur le capteur 137 avant la remontée maximale du pied concerné parce que la pression fluide a chuté trop rapidement, rien n'est changé dans la position du rochet 71 puisque le moteur 93, l'écrou 95 et la vis 96 l'ont placé dans une position axiale débloquée irréversible.

Lorsque l'un des pieds de l'individu dépasse l'autre, l'aspérité de la piste 139 établi un contact double 157 et 158 qui envoie dans le moteur 93 une impulsion de courant continu de sens contraire à celle qui lui a été envoyée par le capteur 137, donc l'articulation 4 est replacée en position de blocage possible.

On peut aussi utiliser dans ce cas le moyen mécanique de déblocage décrit sur la figure 38 à condition que le circuit de commande de la figure 53 soit perfectionné par un relais électromagnétique du type de celui qui sera décrit pour la figure 55 et qui permet au contact établi par le capteur 137 de se maintenir en l'absence de force de poussée jusqu'à ce que le contact soit coupé dans le capteur 138a.

La deuxième fonction à remplir par les éléments du système automatique de pilotage est la vidange et l'alimentation en fluide des volumes étanches déformables contenus dans les dispositifs téléscopiques 3.

Dans le cas des applications les plus courantes de l'appareil selon l'invention, la vidange des dispositifs téléscopiques débute à la fin de la foulée propulsive de l'individu, correspondant donc à la fin de l'opération décrite sur les figures 16 et 20, et donc au voisinage de l'expansion maximale des dispositifs téléscopiques 3.

Dans la plupart des cas, la détection de cette position d'expansion maximale faite par le capteur 137 fera double emploi, pour la vidange des dispositifs téléscopiques 3 et pour le déblocage de l'articulation 4 bien que les circuits relatifs à ces opérations aient été discernés pour favoriser l'explication. Il y a cependant des cas où il pourra être nécessaire d'avoir deux capteurs 137 dont l'un détectera un signal légèrement antérieur à la position d'extension maximale du dispositif téléscopique.

L'alimentation automatique en fluide sous pression des dispositifs téléscopiques dépend beaucoup, contrairement à la vidange, du mode d'utilisation de l'appareil. On a vu plus haut que l'alimentation est effectuée par le distributeur 105 ou 105a, au moyen de deux tiroirs 106 et 107 dont le fonctionnement a été expliqué : le déplacement du tiroir 106 s'obtient par envoi du courant dans l'un des solénoïdes 108 ou 109 qui causent le déplacement axial maximal du tiroir 107, lequel entraîne le tiroir 106 et le place en position de vidange de l'un des dispositifs téléscopiques. L'admission ne peut avoir lieu dans l'autre dispositif téléscopique que lorsque le courant est coupé dans l'électro-aimant concerné.

Par conséquent, si un capteur 137 appartenant par exemple au côté droit de l'appareil selon l'invention, établit le contact dans l'électro-aimant 108 pour vidanger le téléscope droit, c'est du capteur 138b du côté droit et de la piste 139 du côté droit que dépendra la coupure du courant dans l'électro-aimant 108 donc l'admission de fluide dans le téléscope gauche.

L'individu utilisateur de l'appareil peut se déplacer ainsi que l'indiquent les figures 12 à 17, c'est-à-dire selon un mode bondissant plus ou moins accentué effectué à plat ou en côte. Il peut aussi se déplacer ainsi que l'indiquent les figures 18 à 20, c'est-à-dire de telle manière qu'après que le pied ait touché le sol en avant, il y ait nécessité d'alimentation immédiate ou presque immédiate du dispositif téléscopique relié à ce pied. La coupure du courant dans l'électro-aimant 108 ne sera pas due dans ces deux cas d'utilisation au même paramètre physique. Dans le premier cas, la coupure sera due au passage du frotteur 145 du capteur 138b sur une discontinuité d'épaisseur de la piste 139 et dans le second cas, la coupure sera due à l'inversion de sens de frottement du frotteur 145 sur la piste 139.

Ce sont cependant les cas extrêmes et de nombreux cas intermédiaires sont possible pouvant comprendre à chaque fois : une intervention manuelle à l'arrêt avant le départ sur le réglage du pilotage automatique ; une intervention manuelle continue pendant la marche sur le réglage du pilotage automatique ; enfin un pilotage totalement manuel.

Le schéma de fonctionnement de la figure 55 prend en compte toutes ces éventualités intermédiaires et les cas extrêmes.

Pour établir le courant dans le circuit d'alimentation du solénoïde 108 actionnant le tiroir 107, il faut les conditions suivantes :
. Il y a du courant continu envoyé par la source 151.
. Le contact manuel 160 est établi.
. Le capteur 137 est en position de butée de fin de course et son contact est établi.
. Le contact a lieu dans le capteur 138b.

Ainsi le courant passe dans le solenoïde 108 et aussi dans l'électro-aimant relai 161 qui se déplace axialement et établit le contact en 162 et 163. De cette façon, si le contact mécanique cesse sur le capteur 137 et que le courant est coupé, rien ne sera changé dans la suite des opérations. Le courant passera donc dans l'ensemble du circuit jusqu'au moment où il sera coupé par le capteur 138b, ce qui déclenchera l'admission de fluide dans l'autre téléscope, le solénoïde 108 n'étant plus alimenté.

Si l'individu se déplace selon un mode bondissant, la vis de réglage 164 est désserrée vers le bas au maximum de sorte que la mise en contact du frotteur 145 sur la butée 148 ne puisse pas couper le courant car la lame flexible conductrice 149 peut descendre sur son plan incliné d'appui et de contact. Ainsi, il faut attendre que le frotteur 145, venant de la gauche de la figure, donc de la position arrière extrême du dispositif téléscopique qui est en cours de vidange depuis l'établissement du courant, atteigne la discontinuité de la piste 139 et soit au-dessus de la partie la moins épaisse de celle-ci pour que le courant soit coupé. Avec le moyen de correction manuelle du pilotage automatique, composé d'une poignée 144 envoyant avec un circuit électrique séparé des impulsions sur le moteur pas à pas 143, l'individu a le moyen de déplacer la position de la discontinuité de la piste 139. Par conséquent, s'il déplace la piste vers la gauche de la figure, la coupure de courant sera à chaque fois obtenue automatiquement plus tôt, donc l'alimentation du téléscope opposé aura lieu plus tôt, ce qui favorisera par exemple la montée d'une côte en mode bondissant.

Si l'individu se déplace lentement en forte côte ou dans un escalier, il y a nécessité d'alimentation immédiate ou presque immédiate du dispositif téléscopique associé au pied qui vient de toucher le sol. La vis de réglage 164 est avant le départ resserrée vers le haut pour empêcher la lame flexible conductrice 149 de descendre trop bas sur son plan incliné d'appui et de contact. Ainsi, lorsque le capteur 138b qui se dirigeait vers la gauche de la figure en maintenant son frotteur 145 au contact de la lame 149, inverse son mouvement et se dirige vers la droite, le contact est coupé instantanément ou presqu'instantanément selon la longueur de parcours sur le plan incliné que le réglage de la vis 164 autorisera à la lame 149. L'individu peut aussi monter des escaliers ou des pentes très fortes sans ce dernier système et en utilisant le dispositif précédent avec un réglage approprié de la position de la discontinuité de la piste 139 à condition qu'il se déplace à une certaine vitesse donc avec un mouvement de ciseaux des jambes plus net. Le second dispositif n'est destiné qu'à accroître la précision dans certains cas généralement peu fréquents d'utilisation. Par ailleurs, les mouvements de réglage par la vis 164 sont décrits ici très schématiquement et ils peuvent être motorisés pour permettre des interventions manuelles en continue sur le pilotage automatique.

Enfin, il existe un dernier mode de pilotage qui est totalement manuel. On règle à nouveau la vis 164 vers le bas avant le départ de manière à ce que seule la différence d'épaisseur de la piste 139 puisse couper le courant. En marche, on effectue à chaque pas une coupure manuelle prématurée sur le circuit grâce au contacteur manuel 160. Ce mode de pilotage s'applique à des déplacements très lents, pour infirmes montant un escalier par exemple.

La dernière fonction essentielle à piloter ne sera que faiblement évoquée car elle n'est pas caractéristique de l'invention et les moyens de réalisation sont bien connus de l'homme de l'art. L'individu qui utilise l'appareil selon l'invention doit naturellement être maître de la puissane motrice. Le paramètre qui l'intéresse est en principe uniquement la pression moyenne qui règne dans le réservoir tampon 103. C'est d'elle que dépendra la douceur ou la vigueur de l'effort d'extension des dispositifs téléscopiques 3 et par conséquent, l'efficacité du mouvement d'assistance à la propulsion par ses jambes.

Ce paramètre se règle indépendamment des autres aspects du pilotage de l'appareil grâce à une commande manuelle groupée sur la même poignée, que le contacteur 160 et la commande 144 du réglage de l'admission automatique. Cette poignée, tournante dans deux sens, ou/et munie de boutons poussoirs selon le cas, peut être simplement tenue à la main et reliée à l'appareil par un faisceau de fils souples. Elle peut aussi être solidaire de l'appareil comme les poignées de soutènement 180 que l'on verra plus loin. Cette commande qui affiche le désir de l'individu d'une pression plus ou moins élevée dans le réservoir tampon 103 intervient directement sur le régulateur 102 qui asservit la puissance du moteur à la pression moyenne choisie pour le réservoir 103 quel que soit le débit exigé, dans les limites possibles, par les dispositifs téléscopiques 3. L'utilisation de l'appareil est ainsi sans danger puisque l'individu augmente progressivement la pression du réservoir 103 en fonction des besoins et possibilités d'un parcours donné et compte tenu de ses aptitudes physiques.

Les figures 57 à 62 sont relatives à quelques variantes apportées à l'appareil selon l'invention.

Les figures 56 et 57 décrivent un système d'assistance mécanique au mouvement de ciseaux des jambes, c'est-à-dire au mouvement pendulaire des dispositifs téléscopiques 3. Ce système utilise tout l'appareil selon l'invention et constitue donc un simple ajout sur celui-ci sans aucune modification des dispositions précédemment décrites.

Le sommet de chacun des dispositifs téléscopiques 3 est relié par une articulation 167 à un vérin téléscopique 166 dont une articulation opposée 168 est reliée à un point fixe suffisamment solidaire de la selle et situé en arrière de celle-ci. Par exemple, sur la figure 56, l'articulation 168 est fixée à la base du groupe moto-propulseur 7 mais ce n'est pas limitatif. Les vérins 166 possèdent des pistons 169 dont la course est suffisante pour ne pas entraver les mouvements pendulaires complets de chacun des dispositifs téléscopiques 3. L'alimentation en fluide comprimé des vérins 166 est très simple car chacun d'eux est relié à la canalisation d'alimentation du dispositif téléscopique gauche ou droit qui lui est opposé par rapport à la cloison médiane de la selle. Cela permet donc d'obtenir la projection vers l'avant de la jambe qui est en l'air, pendant que la jambe qui est au sol et en arrière effectue la propulsion. Toutefois, la vidange de ces vérins 166 peut devoir s'effectuer dans certains cas d'utilisation sans bondissement, de manière beaucoup plus rapide que celle des dispositifs téléscopiques 3 car ceux-ci bénéficient du rôle des articulations déblocables 4. Par conséquent, les canalisations 169 qui relient chaque vérin 166 à la canalisation d'alimentation du dispositif téléscopique 3 opposé sont munies d'une électro-vanne à trois voies 170 qui permet, soit l'alimentation des vérins 166, soit la coupure de la canalisation d'alimentation 169 et la mise en relation du vérin avec une canalisation 171. Cette canalisation 171 est reliée à l'atmosphère si le fluide de propulsion est de l'aire comprimé, et elle est reliée à une soupape 172 située à l'entré de l'autre verin 166 si le fluide de propulsion est un liquide. Ainsi, l'effort mécanique résistant exercé par les vérins 166 pendant leur vidange est négligeable. La commande des électro-vannes 170 est effectuée grâce à un contact établi par le capteur 137 précédemment décrit.

Il est souhaitable que, lorsque l'appareil selon l'invention est muni des vérins 166, il comprenne aussi les trois roues côniques 25d, 25g et 26 qui assurent le maintien de la position angulaire de la selle 1.

Cependant, si les vérins 166 sont à double effet, la face 174 des pistons 173 peut exercer une pression sur un liquide qui fait communiquer les deux vérins 166 par la canalisation 175. Ainsi, l'avance d'un des pistons 173 est obligatoirement accompagnée d'un recul identique de l'autre piston 173, ce qui donne une certaine stabilisation angulaire entre la selle et la bissectrice de l'angle formé à tout moment par les deux dispositifs téléscopiques 3.

Les figures 58 et 59 décrivent en vue de profil et en vue de dos, l'appareil selon l'invention équipant un individu. L'appareil possède dans ce cas deux types d'appuis supplémentaires avec le corps, ces deux éléments pouvant être employés indépendamment l'un de l'autre.

L'appui normal transmettant les efforts ascendants produits par l'appareil est la selle 1. Ces efforts concernent donc le bas de la colonne vertébrale de l'individu. Les moyens décrits sur les figures 58 et 59 sont destinés à concerner aussi le haut de la colonne vertébrale par une action ascendante exercée soit sur les mains, soit sous les épaules, soit aux deux endroits à la fois. L'avantage est, pour une personne normale, de répartir les efforts reçus en plusieurs points au lieu d'un seul ce qui peut augmenter le confort surtout si la puissance de l'appareil est elevée et, pour un infirme, de pallier une déficience de la colonne vertébrale. Par ailleurs, le fait d'utiliser un appui aux épaules relié par une barre rigide à la selle 1 permet de donner, si besoin est pour certains usages, des degrés de stabilisation supplémentaires à la selle par rapport au corps. La suspension sous les épaules comprend un moyen d'appui 176 qui peut être une barre en métal ou plastique à section, plus ou moins applatie, pour bien s'adapter à la forme du dos et se terminer par des parties concaves vers le haut passant sous les épaules. La pièce 176 pourrait également aboutir au-dessus des épaules et soutenir celles-ci par des courroies passant au-dessous des bras ou tout autre moyen approprié. L'ensemble peut aussi être complété par une courroie passant devant la poitrine pour augmenter la stabilité.

La pièce 176 possède, en général en son milieu, une articulation 177 dont l'axe est sensiblement parallèle au sol et longitudinal par rapport au corps. Cette articulation peut être bloquée pour certains usages.

Une barre de liaison 178 métallique ou en plastique à section éventuellement très applatie et élargie pour bien s'adapter à la forme du dos, descend contre celui-ci jusqu'à une articulation 179 fixée à l'arrière de la selle 1. Cette articulation comprend un axe longitudinal par rapport à la selle et au corps et sensiblement parallèle au sol et un axe perpendiculaire au plan médian de la selle.

Avec ces deux axes, l'articulation 179 permet à la barre 178 de n'exercer aucun couple sur la selle mais comme il est dit plus haut, il peut être avantageux pour certains usages de l'appareil que la barre 178 puisse exercer un couple sur la selle pour la maintenir en position. Par conséquent les deux axes de l'articulation 179 sont blocables, simultanément ou séparément. Les moyens de blocage possibles sont suffisamment connus de l'homme de l'art pour ne pas avoir à être décrits.

La suspension par les mains comprend deux poignées 180 disposées symétriquement par rapport au corps à quelques centimètres à l'extérieur des cuisses. L'axe de ces poignées 180 est sensiblement parallèle au sol lorsque l'appareil est monté sur l'individu qui est debout et ces poignées sont sensiblement parallèles entre elles. Elles sont solidement reliées à l'arrière de la selle par des tubes galbés métalliques ou plastiques 181 qui contournent l'arrière des cuisses de l'individu en laissant le libre débattement de celles-ci. Les tubes galbés 181 sont fixés sur la structure résistante de l'arrière de la selle par un moyen qui permet également un réglage de position angulaire autour d'un axe perpendiculaire au plan médian de la selle. Ce moyen peut être composé par exemple d'un écrou à collet qui vient bloquer contre la pièce filetée mâle, solidaire de la selle, un collet à denture radiale pratiqué sur l'extrêmité de chaque tube 181. Ainsi, l'individu peut adapter la hauteur des poignées à la longueur de ses bras pour être dans la position la plus favorable à la transmission par ceux-ci d'un effort vertical important.

L'une des poignées ou les deux peuvent jouer de plus le rôle de commandes de pilotage comme une poignée de moto si l'appareil comprend un groupe moto-compresseur.

Les figures 60 et 61 décrivent deux variantes du dispositif téléscopique 3 selon l'invention. Elles concernent uniquement la manière de rendre possible le coulissement en présence d'un couple mais les autres fonctions de ces dispositifs téléscopiques, c'est-à-dire l'application d'une force d'extension par un ressort ou un fluide et les ressorts de rappel sont réalisées par adaptation des principes déjà décrits et ne seront pas répétées. De plus, les articulations sur la selle et l'articulation blocable et déblocable 4 sont également identiques à celles du dispositif téléscopique 3 nominal ainsi que les moyens de mise en oeuvre du pilotage.

Sur la figure 60 on distingue une barre métallique rigide 182 partant des articulations de la selle et aboutissant à une articulation 183 à axe sensiblement perpendiculaire au plan médian du corps. L'articulation 183 est reliée à une plaque métallique 184 qui porte l'articulation blocable et déblocable 4 et une articulation 185 à axe sensiblement perpendiculaire au plan médian du corps. L'articulation 185 est reliée à une partie coulissante mâle ou femelle d'un vérin téléscopique 186 dont l'autre partie coulissante mâle ou femelle est reliée par une articulation 187 à axe sensiblement perpendiculaire au plan médian du corps à la barre 182.

Sur la figure 61 on distingue une barre métallique rigide 188 partant des articulations de la selle et aboutissant à une articulation 189 à axe sensiblement perpendiculaire au plan médian du corps. L'articulation 189 est reliée à une plaque métallique 190 qui porte l'articulation blocable et déblocable 4 et une articulation 191 à axe sensiblement perpendiculaire au plan médian du corps. L'articulation 191 est reliée à une partie coulissante mâle ou femelle d'un vérin téléscopique 192 dont l'autre partie coulissante mâle ou femelle est reliée par une articulation 193 à axe sensiblement perpendiculaire au plan médian du corps à la barre 188.

Ainsi, on constate que les barres 182 ou 188 et les plaques 184 ou 190 auxquelles elles sont articulées sont un moyen de remplacer les roulettes des dispositifs téléscopiques 3 précédemment décrits et qu'elles permettent d'obtenir l'effort de propulsion sous l'effet de l'expansion du vérin téléscopique 186 ou du raccourcissement du vérin téléscopique 192 sans que ceux-ci aient à subir un couple. Ces deux procédés qui mettent en jeu des forces plus importantes dans les vérins nécessitent des sections plus grandes pour ceux-ci que pour ceux décrits jusqu'à présent et ils sont relativement encombrants.

En revanche, et bien qu'il soit possible d'équiper les dispositifs téléscopiques 3 nominaux, munis de roulettes, de vérins à double effet permettant de descendre le pied et aussi de la remonter avec une action mécanique pilotée, les vérins téléscopiques 186 ou 188 sont plus facilement adaptables à cet usage, destiné par exemple à certains infirmes. Pour certaines de ces applications de l'appareil selon l'invention nécessitant des vérins à double effet, capables de permettre aussi l'assistance à la remontée du pied, il est possible de remplacer l'articulation blocable et déblocable 4 par une fixation rigide du bras de jonction 5 sur le dispositif téléscopique car l'utilisation ne comprend alors en général que des mouvements sufisamment lents. Il est également possible de bloquer l'articulation 6 de manière à rendre la chaussure solidaire du bras 5.

La figure 62 représente un moyen supplémentaire pour permettre la rotation du pied autour de l'axe des jambes avec l'appareil selon l'invention.

Le bras de jonction 5 est muni d'une articulation 194 à axe sensiblement perpendiculaire au sol et qui se trouve à proximité de l'articulation 4. Ceprocédé peut permettre de faire des choix techniques plus larges pour les dispositifs téléscopiques 3 à roulettes des figures 29 à 34, s'ils n'ont plus l'obligation d'assurer une rotation mutuelle des parties coulissantes autour de leur axe longitudinal. Il peut s'appliquer en particulier, ainsi que tout autre procédé jouant le même rôle et fixé à la base ou au sommet des dispositifs téléscopiques, au cas où le coulissement n'est pas rectiligne ou au cas des dispositifs téléscopiques des figures 60 et 61.

Il existe un certain nombre de modes d'apports particuliers de l'énergie et de variantes de l'appareil lorsque celui-ci ne nécessite qu'une très courte autonomie. Les usages auxquels il est alors réservé de préférence sont l'ascension répétitive d'une pente forte ou d'un escalier dont les dénivelées n'excèdent pas quelques dizaines de mètres et en général, sur une distance de quelques dizaines de mètres également. Les applications peuvent concerner le travail dans certaines mines ou sur un chantier de bâtiment ou travaux publics par exemple, ou encore la montée, par des infirmes ou diminués physiques, d'escaliers dans lesquels il est difficile ou trop coûteux d'installer un système quelconque d'ascenceur.

La première solution consiste à brancher un réservoir d'air comprimé portable à très haute pression, muni d'un détenteur, directement sur la canalisation 113 de la figure 50. L'énergie de détente n'est alors pas récupérée et l'évacuation se fait à l'air libre par la canalisation 130. L'autonomie de dénivelée est courte et elle dépend de la proportion du travail mécanique de propulsion qui est demandée à l'appareil par rapport au travail fourni par les jambes. En revanche, l'autonomie en distance parcourue n'est pas limitée.

La deuxième solution consiste à alimenter la canalisation 113 de la figure 50 par un long et fin tuyau souple qui amène en permanence de l'air comprimé provenant d'une source fixe. Les sources d'air comprimé sont très répandues dans les usines ou les chantiers divers de sorte qu'il est facile à l'ouvrier porteur de l'appareil et devant effectuer localement un travail pénible d'ascension, de brancher son tuyau souple à proximité. L'autonomie en dénivelée et en distance parcourable est relativement faible mais le travail demandé peut ainsi se poursuivre indéfiniment. Ce moyen peut être avantageusement utilisé par les diminués physiques pour gravir un escalier car il est facile de faire descendre le tuyau souple d'air comprimé au milieu de la cage d'escalier aussi petite soit elle et à priori quel que soit le nombre d'étages. On verra plus loin comment l'appareil peut être rapidement positionné sur le corps pour cet usage particulier. Ainsi, le diminué physique, capable par exemple de marcher, comme un grand cardiaque, mais non capable de monter un escalier, pourra laisser constamment un appareil personnel réservé à cet usage au voisinage de l'escalier qu'il aura à emprunter souvent.

La troisième solution consiste à équiper l'appareil d'un groupe moto-compresseur à moteur électrique et à alimenter celui-ci par un fil souple, dans des conditions identiques aux cas d'emploi d'un tuyau souple d'air comprimé.

La quatrième solution, qui fait appel à l'énergie électrique, apportée par un fil souple, ou pour une très faible autonomie, par une batterie portable, nécessite une variante technologique sur le mode d'exercice de la force d'extension dans les dispositifs téléscopiques 3. L'explication, très simple, se réfère aux dessins existants.

Sur la figure 33, on conserve le piston 55 et sa rallonge inférieure 57. On supprime le ressort de rappel ·61 et sa plaque d'appui 58. On diminue le diamètre de la vis 50 pour dégager le centre du tube fendu 57. On fixe au sommet du piston 55 un écrou à circulation de bille, bien connu de l'homme de l'art. Une tige filetée dont l'axe est confondu avec celui de la partie mâle ou supérieure 3s du dispositif téléscopique peut tourner dans cet écrou à billes et elle traverse le sommet de la partie 3s. Cette traversée s'effectue par un palier à bille qui lui permet de tourner et d'exercer des efforts axiaux importants. Après la traversée du sommet de la partie 3s, la tige filetée est fixée sur une roue dentée cylindrique faisant partie d'un réducteur à engrenages parallèles dont le dernier est solidaire d'un moteur électrique. L'axe de ce moteur est donc paralèle à celui du dispositif téléscopique 3s et à une distance telle que l'encombrement extérieur du moteur permette juste le passage de la partie femelle ou inférieure 3i du dispositif téléscopique lorsqu'il coulisse. Ainsi le moteur électrique n'est donc fixé que par son extrêmité supérieure au réducteur à engrenages parallèles, lui-même fixé à l'extrêmité supérieure de la partie 3s du dispositif téléscopique. La disposition de ce moteur, en-dessous du niveau du sommet de la partie 3s n'augmente pas l'encombrement vertical de celui-ci à la faible épaisseur près du réducteur à engrenages parallèles. La course pendulaire des dispositifs téléscopiques 3 reste donc suffisante.

Ce mode d'exercice de la force d'extension des dispositifs téléscopiques 3 nécessite donc un moteur électrique gauche et un droit, mais tous les dispositifs représentés sur les figures 46 et 50 sont supprimés. Les moteurs, les vis et écrous à billes effectuent les mouvements d'extension et de raccourcissement des dispositifs téléscopiques. Les types de pilotage possibles sont directement dérivés de ceux décrits plus haut et représentés sur les figures 51 à 55.

L'appareil objet de l'invention muni de la variante ci-dessus ne s'applique plus qu'à des mouvements de propulsion relativement lents mais qui conviennent cependant pour l'ascension des escaliers par les diminués physiques ou pour l'assistance à certains travaux pénibles.

Pour des usages très ponctuels de l'appareil selon l'invention tels que la montée de quelques étages avec un escalier, il est possible de positionner celui-ci sur le corps de façon très sommaire et très rapide : les articulations 6 sont alors fixées au sommet de cavités rigides à parois minces en tôle ou en plastique dans lesquelles on peut enfoncer le bout des chaussures qui vient s'y coincer légèrement grâce à un revêtement intérieur en caoutchouc mousse de ces cavités par exemple. L'appareil se comporte alors à peu près comme si les articulations 6 étaient reliées de façon rigide avec la chaussure. Par ailleurs, la selle 1 ne possède alors pas de dispositif de fixation 2 sur les hanches. Elle est par contre muni des deux poignées latérales 180 qui sont suffisantes pour la maintenir au contact du corps pendant un temps court. Ainsi, l'utilisateur pressé doit seulement enfoncer ses deux points de chaussures dans les cavités reliées à l'articulation 6 et placer la selle sous ses fesses en tenant les poignées 180 et il peut démarrer aussitôt. Les cavités reliées à l'articulation 6 ne sont pas limitatives de cet usage rapide de l'invention et d'autres types d'accrochage sommaires de l'articulation 6 avec une chaussure ordinaire peuvent évidemment être imaginés.

L'appareil selon l'invention peut aussi fonctionner sans moteur comme un siège à position verticale indifférente permettant d'effectuer certains travaux de peinture, etc ... On utilise alors la transmission d'efforts par liquide. Un réservoir 103 contient, derrière une vessie souple 129 ou un système de pistons, une quantité de gaz se trouvant pour la température et les pressions concernées dans des conditions proches de son équilibre liquide-gaz. De ce fait, le changement de volume de celui-ci s'accompagne d'une faible variation de pression, ce qui permet d'exercer sur un piston contenu dans le dispositif téléscopique 3 une force relativement constante tout au long de la course de celui-ci et de disposer d'un siège à hauter indifférente.

Comme il a été dit plusiers fois pour des aspects particuliers, l'invention n'est pas limitée aux seules formes ou moyens décrits et représentés. Des formes équivalentes, ou des moyens différents mais évidents pour l'homme de l'art et dont la mise en oeuvre n'apporterait pas une réelle activité inventive, font partie de l'invention dont la portée est précisée par les revendications suivantes.

## Revendications

1. Appareil d'assistance mécanique de la propulsion par les jambes, caractérisé en ce qu'il comporte, en combinaison, une selle (1) munie de moyens de fixation s'adaptant, seuls ou en association, aux hanches, aux épaules ou aux mains, ladite selle comportant une articulation (11) d'axe sensiblement perpendiculaire à son plan médian, à laquelle sont reliées l'une des extrêmités de deux dispositifs téléscopiques (3), situés de part et d'autre dudit plan médian, l'autre extrêmité de chacun de ces dispositifs téléscopiques (3) comportant une articulation (4), d'axe sensiblement perpendiculaire au leur, munie d'un moyen de blocage et de déblocage automatique, ladite articulation (4) étant reliée sensiblement perpendiculairement à une extrêmité d'un bras de jonction (5) dont l'autre extrêmité comporte une articulation (6) sensiblement parallèle à l'articulation (4) qui le relie à un accessoire fixé sur l'avant d'une chaussure de manière à ce que l'axe de l'articulation (6) soit sensiblement parallèle à la semelle de la chaussure et sensiblement perpendiculaire à sa plus grande dimension, la forme du bras de jonction (5) lui permettant de tourner librement autour de l'articulation (6) sans heurter la chaussure et les deux dispositifs téléscopiques (3) étant munis de moyens moteurs associés pour assister, l'articulation (4) étant bloquée, l'effort musculaire de propulsion par les jambes.

2. Appareil d'assistance mécanique de la propulsion par les jambes selon la revendication 1, caractérisé en ce que l'articulation (11) de la selle est complétée par une articulation très voisine (12) à axe sensiblement parallèle au plan médian du corps, qui lui est reliée par une pièce de raccordement (16) ou (32) et qui permet d'écarter latéralement par rapport audit plan médian les dispositifs téléscopiques (3), l'ordre, en allant de la selle (1) vers les dispositifs téléscopiques (3) de l'articulation (11) à axe perpendiculaire et de l'articulation (12) à axe parallèle audit plan médian, pouvant être inversé et l'articulation (12) possédant une butée angulaire (28) ou (33) qui interdit aux dispositifs téléscopiques (3) de traverser ledit plan médian, donc de se heurter.

3. Appareil d'assistance mécanique de la propulsion selon l'une quelconque des revendications 1 et 2, caractérisé en ce qu'un secteur de roue dentée conique (25) solidaire en rotation de chacun des deux dispositifs téléscopiques (3) et centré sur l'articulation (11) de la selle, coopère avec une roue dentée conique (26) tournant librement sur un axe situé dans ledit plan médian et solidaire de la selle, de telle sorte que pendant les mouvements pendulaires des dispositifs téléscopiques (3) autour de l'articulation (11), l'angle formé par la bissectrice de l'angle variable existant entre eux, avec l'axe de la roue dentée conique (26) reste constant.

4. Appareil d'assistance mécanique de la propulsion selon l'une quelconque des revendications 1 à 3 caractérisé en ce que la dispositif téléscopique (3) est composé d'au moins un élément mâle (3s) et un élément femelle (3i) pouvant coulisser l'un dans l'autre, même en présence d'un couple exercé par le bras de jonction (5), grâce à des roulettes (42), (43), (44) et (45), (46), - (47), ou (50), (51), ou à des billes ou à tout autre procédé avec ou sans cage intermédiaire de guidage, un mouvement de rotation mutuelle pouvant s'effectuer autour de l'axe des composants mâle - (3s) et femelle (3i), d'un angle suffisant pour permettre à l'individu d'écarter ou resserrer la pointe du pied, grâce à l'emploi de tubes cylindriques - (3s) et (3i) ou bien d'un tube mâle cylindrique (3s) relié par une articulation axiale à une pièce (53) qui sert de support aux roues de guidage (50) et (51) qui roulent dans un tube femelle (3i) de section non circulaire et le dispositif téléscopique (3) possédant un ressort hélicoïdal de rappel (39) ou - (61) dont le rôle est de faire rentrer la partie mâle - (3i) dans la partie femelle (3s).

5. Appareil d'assistance mécanique de la propulsion selon l'une quelconque des revendications 1 à 4 caractérisé en ce que le moyen pour exercer, dans les dispositifs téléscopiques (3), la force destinée à les allonger est soit un ressort (48) préalablement comprimé lors du raccourcissement desdits dispositifs téléscopiques, soit une vis axialement disposée et axialement immobilisée dans la partie mâle (3i) ou femelle (3s) coopérant avec un écrou solidaire de l'autre partie, soit une chambre étanche déformable pouvant recevoir un fluide sous pression et permettant à celui-ci d'exercer une force motrice sur les extrêmités de la chambre solidaires respectivement de la partie mâle (3i) et femelle (3s).

6. Appareil d'assistance mécanique de la propulsion selon l'une des revendications précédentes caractérisé en ce que la puissance motrice extérieure destinée à l'expansion des dispositifs téléscopiques (3) provient, dans le cas où la force s'exerce par l'action d'un fluide sous pression, soit d'un groupe moto-compresseur portable par l'individu, soit d'un réservoir portable de gaz comprimé, tous deux reliés à la selle (1) par des canalisation souples et démontables, soit d'une canalisation souple de fluide sous pression provenant d'un générateur fixe et dans le cas où la force s'exerce par un système vis et écrou contenu dans chaque dispositif téléscopique (3), par un moteur électrique fixé sur chacun de ceux-ci au voisinage de la selle (1) et entraînant en rotation au moyen d'un réducteur, la vis ou éventuellement l'écrou.

7. Appareil d'assistance mécanique de la propulsion selon l'une quelconque des revendications précédentes caractérisé en ce que l'articulation (4) permet à l'ensemble du dispositif téléscopique (3) et du bras de jonction (5) partant d'une position repliée, de se déplier librement jusqu'à l'ouverture angulaire maximale autorisée par une butée ou jusqu'à une ouverture moindre mais ne permet pas le parcours angulaire inverse grâce à la présence d'un rochet (71), l'action de ce dernier étant cependant supprimée automatiquement lorsque l'expansion du dispositif téléscopique (3) devient voisine du maximum et ce rochet étant automatiquement remis en service lorsque, s'étant suffisamment raccourci en service, le dispositif téléscopique (3) recommence à s'allonger, ce qui correspond sensiblement au point de croisement mutuel des dispositifs téléscopiques tournant autour de l'articulation (11) de la selle (1).

8. Appareil d'assistance mécanique de la propulsion selon les revendications 1 à 6, caractérisé en ce que pour certains usages à basse vitesse, l'articulation (4) peut être bloquée en permanence et, dans ce cas, le moyen qui exerce une force pour l'allongement du dispositif téléscopique (3) est en mesure d'en exercer aussi une pour le raccourcissement de celui-ci.

9. Appareil d'assistance mécanique de la propulsion selon la revendication 1 caractérisé en ce que les dispositifs téléscopiques (3) comportent un vérin téléscopique travaillant généralement en extension (186) ou en traction (192) dont le coulissement en présence d'un couple exercé par le bras de jonction (5) est rendu possible dans le premier cas par une barre rigide (182) reliée aux articulations (11), (12) de la selle, articulée à une extrêmité (187) du vérin téléscopique (186) et en un point (183) d'une plaque (184) reliée à l'articulation (4) et articulée à l'autre extrêmité (185) de ce vérin téléscopique (186), et dans le second cas par une barre rigide (188) reliée aux articulations (11) (12) de la selle articulée à une extrêmité (193) du vérin téléscopique (192) et en un point (189) d'une plaque (190) reliée à l'articulation (4) et articulée à l'autre extrêmité (191) de ce vérin téléscopique (192), toutes les articulations ayant leur axe sensiblement perpendiculaire au plan formé par le vérin (186) ou (192) et la bras de jonction (5).

10. Appareil d'assistance mécanique de la propulsion selon l'une quelconque des revendications précédentes caractérisé en ce qu'un point voisin de l'extrêmité proche de la selle (1) de chacun des dispositifs téléscopiques (3) est relié par une articulation (167) à un vérin téléscopique (166) dont l'articulation opposée (168) est reliée à un point suffisamment solidaire de la selle et situé en arrière de celle-ci, les vérins (166) étant symétriques par rapport au plan médian de la selle, et possèdant chacun un cylindre intérieur formant étanchéité avec un piston (173) capable d'exercer sous l'action de la pression d'un fluide sur une de ses faces une force poussant le dispositif téléscopique (3) concerné vers l'avant simultanément avec un déplacement de la jambe, les autres faces (174) des pistons (173) et les volumes qu'elles génèrent dans le cylindre intérieur des vérins (166) pouvant être mis en relation par une canalisation (175) afin que la poussée vers l'avant par un vérin (166) sur l'un des dispositifs téléscopiques (3) soit accompagnée d'une traction vers l'arrière par l'autre vérin (166) sur l'autre dispositif téléscopique (3) ce qui est un des moyens de conserver une stabilité angulaire moyenne de la selle par rapport aux jambes.

Fig. 1
Fig. 2
Fig. 3
Fig. 4
Fig. 5
Fig. 6
Fig. 7
Fig. 8
Fig. 9
Fig. 10
Fig. 11

Fig. 12

Fig. 13

Fig. 14

Fig. 15

Fig. 16

Fig. 17

Fig. 18

Fig. 19

Fig. 20

Fig.24

Fig.21

Fig.22

Fig.23

0 229 537

Fig. 28

Fig. 25

Fig. 26

Fig. 27

Fig. 29

Fig. 32

Fig. 30

Fig. 31

Fig.33

Fig.34

Fig.35

Fig.36

Fig. 38

Fig. 37

Fig. 39

Fig 40

Fig.43

Fig.45

Fig.42

Fig.44

Fig.41

Fig.46

Fig.47

Fig.49

Fig 48

Fig. 50

Fig 52

Fig. 53

Fig. 54

Fig. 51

Fig 55

Fig. 57

Fig 56

Fig 62

Fig. 58

Fig 59

Fig. 60

Fig. 61

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A- 247 482 (FABIANI)<br>* En entier *<br><br>--- | 1,4,5 | A 61 H 1/02<br>A 63 B 29/00<br>A 61 H 3/00 |
| A | DE-A- 846 891 (GRISONI)<br>* Revendications 1-4; figures 1-3 *<br><br>--- | 2,3 | |
| A | DE-A-3 227 359 (BOCK)<br>* Page 9, ligne 2 - page 12, ligne 3; figures 1-5 *<br><br>----- | 7 | |

DOMAINES TECHNIQUES
RECHERCHES (Int. Cl.4)

A 61 H
A 63 B
A 61 F

Le présent rapport de recherche a été établi pour toutes les revendications

| Lieu de la recherche | Date d'achèvement de la recherche | Examinateur |
|---|---|---|
| LA HAYE | 28-01-1987 | SCHOENLEBEN J.E.F. |